# EUROPEAN PATENT APPLICATION

(11) **EP 4 840 451 A2**
(43) Date of publication of application: **18.11.2026**
(21) Application number: 26208392.6
(22) Date of filing: 20.09.2024
(51) Int. Cl.: A61K 31/428

(54) **METHOD OF MAKING LANIFIBRANOR**

(30) Priority: 22.09.2023 EP 23306579
(62) Document number(s) of the earlier application(s) in accordance with Art. 76 EPC: 24772702.7 / 4 780 541
(71) Applicant: Inventiva, 21121 Daix (FR)
(72) Inventor: BELL, Frederic, 21121 DAIX (FR); BROSSEAU, Cecile, 21121 DAIX (FR); ZUBAR, Viktoriia, 65929 FRANKFURT AM MAIN (FR)
(74) Representative: Cabinet Beau de Loménie

(57) **Abstract**

The present disclosure relates to a process for manufacturing of a crystalline form of lanifibranor which comprises:
a) preparing a hot slurry of lanifibranor in a suitable solvent for lanifibranor;
b) optionally, filtering the hot slurry of lanifibranor;
c) cooling the slurry of lanifibranor to a temperature from about 15°C to about 95°C; and
d) maintaining the slurry of lanifibranor to a temperature from about 15°C to about 95°C for about 15 minutes to about 72h.

## Description

### Field of the invention

The present disclosure relates to a process for the manufacture of lanifibranor. In particular, the present disclosure relates to a process giving the desired compound in high yield and purity, which can then be incorporated into a pharmaceutical composition.

### Background of the invention

Lanifibranor or 1-(6-benzothiazolylsulfonyl)-5-chloro-1H-indole-2-butanoic acid is a pan-PPAR agonist which is currently in clinical development for the treatment of patients with non-alcoholic steatohepatitis (NASH), for which there is currently no approved therapy.

Processes for manufacturing lanifibranor have been reported (J. Med. Chem. 2018, 61, 6, 2246-2265; WO 2007/026097). However, such processes are laboratory-scale processes which are not suitable for industrial manufacturing and may lead to the formation of by-products and/or impurities which must not be present in pharmaceutical forms intended to be administered to patients. Moreover, such processes use non-recyclable catalysts and the reaction takes a long time for completion (16-30 hours).

There still remains a need for a process for the manufacture of lanifibranor which can be scaled-up, is economical and environmentally friendly in terms of raw materials used, and leads to the desired product with a high yield and purity.

### Summary of the invention

In one aspect the present disclosure relates to a process for the manufacture of lanifibranor, comprising reacting N-(4-chloro-2-iodo-phenyl)-1,3-benzothiazole-6-sulfonamide with 5-hexynoic acid in the presence of Pd/C and a copper salt.

In another aspect, the present disclosure relates to a process for the manufacture of crystalline form beta of lanifibranor, comprising preparing a hot slurry of lanifibranor in a suitable solvent for lanifibranor, and cooling and maintaining the slurry to a temperature from about 20°C to about 85°C for a certain period of time.

### Description of the figures

Figure 1 shows the PXRD pattern of crystalline form beta of lanifibranor.
Figure 2 shows the DSC curve of crystalline form beta of lanifibranor.
Figure 3 shows the TGA curve of crystalline form beta of lanifibranor.
Figure 4 shows the DVS isotherm plot of crystalline form beta of lanifibranor.
Figures 5A and 5B shows the IR spectrum of crystalline form beta of lanifibranor and the indexation of the absorption bands for the IR analysis.
Figures 6 and 7 show the superimposition of PXRD patterns of crystalline forms of lanifibranor.
Figure 8 shows the ¹H NMR spectrum of crystalline form beta of lanifibranor of example 18.
Figure 9 shows the ¹³C NMR spectrum of crystalline form beta of lanifibranor of example 18.
Figure 10 shows the Raman spectrum of crystalline form beta of lanifibranor.

### Detailed description of the invention

As used herein, the term "lanifibranor" is understood to mean lanifibranor free acid, i.e. the compound of formula:

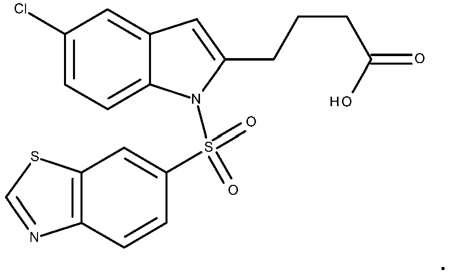

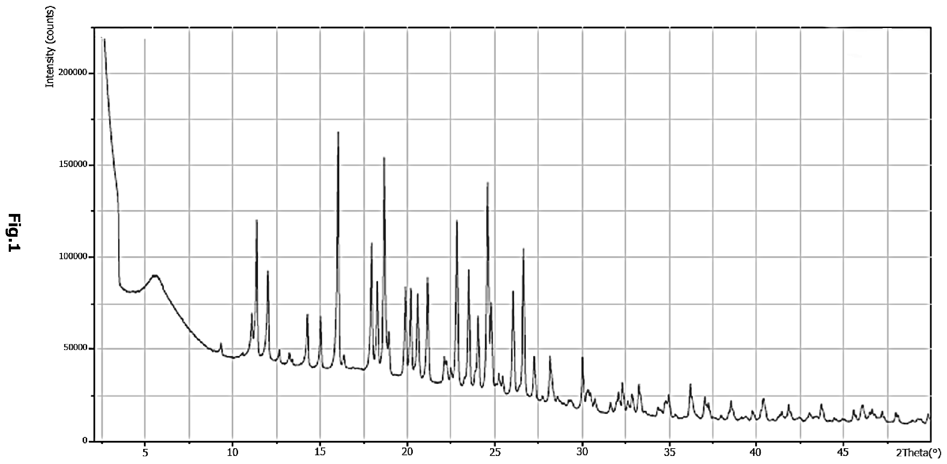

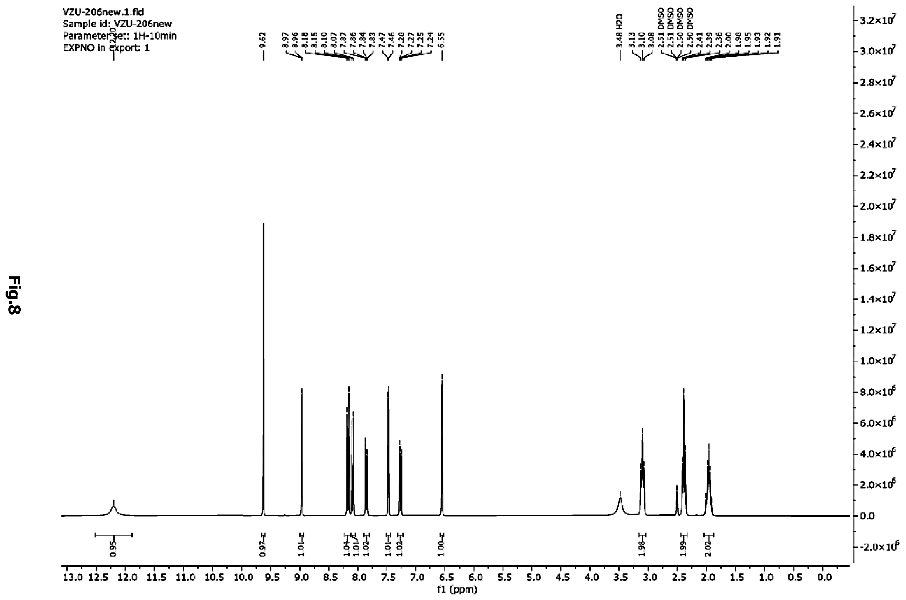

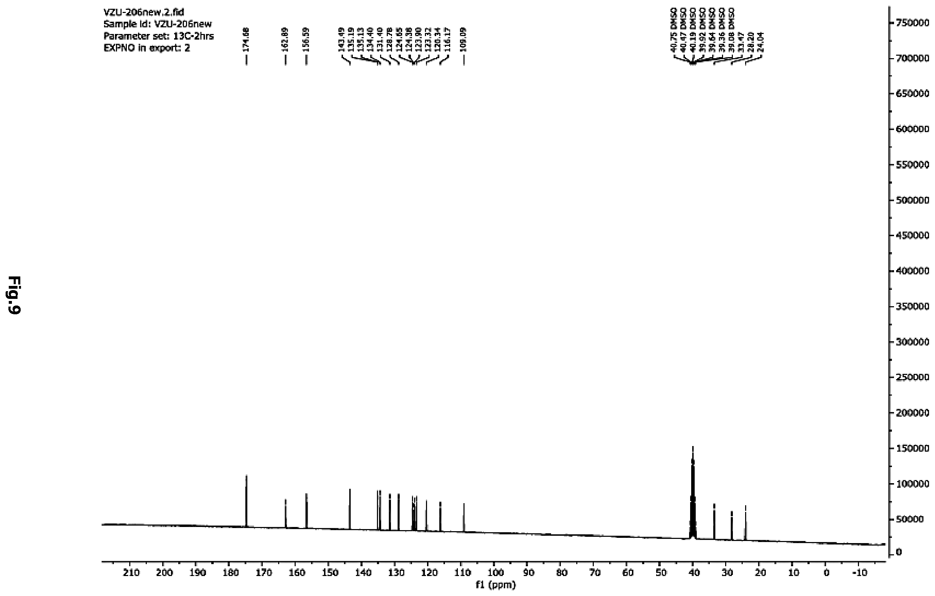

As used herein, "crystalline form beta of lanifibranor" refers to the crystalline form having the characteristics shown in figures 1-10 and in Table 1 below.

As used herein, a "deuterated form of lanifibranor" refers to a compound of formula: 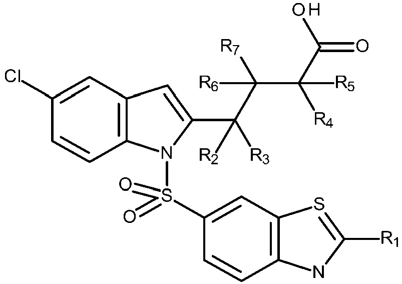 wherein at least one of the groups R₁ to R₇ is a deuterium (D) atom and the other groups R₁ to R₇ are hydrogen (H) atoms. In some embodiments, at least group R₁ is D. In some embodiments, at least one of the groups R₂ to R₇ is D, notably at least one of the groups R₂ and R₃ and/or at least one of the groups R₄ and R₅ and/or at least one of the groups R₆ and R₇ is D. In some embodiments, each of R₂, R₃, R₄, R₅, R₆ and R₇ is D. In some embodiments, the deuterated form of lanifibranor is 4-(1-(2-deuterio-1,3-benzothiazol-6-yl)sulfonyl)-5-chloro-1H-indol-2-yl)butanoic acid or 4-[1-(1,3-benzothiazol-6-ylsulfonyl)-5-chloro-indol-2-yl]-2,2,3,3,4,4-hexadeuteriobutanoic acid.

As used herein, the range "from A to B" includes the end points A and B.

As used herein, all numbers disclosed herein are approximate, whether or not the word "about" or "approximately" is used, and there may be a +/-1%, +/-2%, or +/-5% difference in each number based on the disclosed number.

As used herein, "room temperature" or "ambient temperature," often abbreviated as "RT" means that the temperature of the thing is close to, or the same as, that of the space, e.g., the room or fume hood, in which the thing is located. Typically, room temperature is about 20°C ± 5°C.

The term "suitable solvent for lanifibranor" may be a single solvent or a mixture of solvents, meaning that the solubility of lanifibranor in the single solvent or mixture of solvents is greater than 0.1 g/L, or greater than 0.2 g/L, or greater than 0.3 g/L, or greater than 0.4 g/L, or greater than 0.5 g/L, or greater than 0.6 g/L, or greater than 0.7 g/L, or greater than 0.8 g/L, or greater than 0.9 g/L, or greater than 1 g/L, or greater than 1.1 g/L, or greater than 1.2 g/L, or greater than 1.3 g/L, or greater than 1.4 g/L, or greater than 1.5 g/L, or greater than 1.6 g/L, or greater than 1.7 g/L, or greater than 1.8 g/L, or greater than 1.9 g/L, or greater than 2g/L, or greater than 3 g/L, or greater than 4 g/L, or greater than 5 g/L, or greater than 6 g/L, or greater than 7 g/L, or greater than 8 g/L, or greater than 9 g/L, or greater than 10 g/L, or greater than 15 g/L, or greater than 20 g/L, or greater than 25 g/L, or greater than 30 g/L, or greater than 40 g/L, or greater than 50 g/L, or greater than 60 g/L, or greater than 70 g/L, or greater than 80 g/L, or greater than 100 g/L at room temperature, as determined by methods well known in the art.

As used herein, the term "Pd/C" means palladium on carbon.

In the context of the present disclosure, the various aspects and/or embodiments described herein can be combined.

In one aspect, the present disclosure relates to a process for the manufacture of lanifibranor or a deuterated form of lanifibranor, said process comprising reacting N-(4-chloro-2-iodophenyl)-1,3-benzothiazole-6-sulfonamide with 5-hexynoic acid in the presence of Pd/C and of a copper salt.

In some embodiments, the copper salt includes, but is not limited to, copper chloride (CuCl₂ or CuCl), copper acetate (Cu(OAc)₂), copper carbonate (CuCO₃), copper sulfate (CuSO₄), copper bromide (CuBr), trifluoroacetic acid copper, copper nitrate (Cu(NO₃)₂), copper hydroxide (Cu(OH)₂), copper oxide (Cu₂O or CuO or CuO₂ or Cu₂O₃ or CuO₂), copper iodide (CuI) and mixtures thereof. In some embodiments, the copper salt is copper iodide.

In some embodiments, the copper salt is used in an amount from about 0.01 to about 0.15 equivalent, based on the amount of starting N-(4-chloro-2-iodo-phenyl)-1,3-benzothiazole-6-sulfonamide. For example, from about 0.01 to about 0.14 equivalent, from about 0.01 to about 0.13 equivalent, from about 0.01 to about 0.12 equivalent, from about 0.01 to about 0.11 equivalent, from about 0.01 to about 0.10 equivalent, from about 0.01 to about 0.09 equivalent, from about 0.02 to about 0.15 equivalent, from about 0.02 to about 0.14 equivalent, from about 0.02 to about 0.13 equivalent, from about 0.02 to about 0.12 equivalent, from about 0.02 to about 0.11 equivalent, from about 0.02 to about 0.10 equivalent, from about 0.02 to about 0.09 equivalent, from about 0.02 to about 0.08 equivalent, from about 0.02 to about 0.07 equivalent, from about 0.02 to about 0.06 equivalent, from about 0.02 to about 0.05 equivalent, from about 0.02 to about 0.04 equivalent, from about 0.02 to about 0.03 equivalent, from about 0.03 to about 0.15 equivalent, from about 0.03 to about 0.14 equivalent, from about 0.03 to about 0.13 equivalent, from about 0.03 to about 0.12 equivalent, from about 0.03 to about 0.11 equivalent, from about 0.03 to about 0.10 equivalent, from about 0.03 to about 0.09 equivalent, from about 0.03 to about 0.08 equivalent, from about 0.03 to about 0.07 equivalent, from about 0.03 to about 0.06 equivalent, from about 0.03 to about 0.05 equivalent, from about 0.03 to about 0.04 equivalent, from about 0.04 to about 0.15 equivalent, from about 0.04 to about 0.14 equivalent, from about 0.04 to about 0.13 equivalent, from about 0.04 to about 0.12 equivalent, from about 0.04 to about 0.11 equivalent, from about 0.04 to about 0.10 equivalent, from about 0.04 to about 0.09 equivalent, from about 0.04 to about 0.08 equivalent, from about 0.04 to about 0.07 equivalent, from about 0.04 to about 0.06 equivalent, from about 0.04 to about 0.05 equivalent, from about 0.05 to about 0.15 equivalent, from about 0.05 to about 0.14 equivalent, from about 0.05 to about 0.13 equivalent, from about 0.05 to about 0.12 equivalent, from about 0.05 to about 0.11 equivalent, from about 0.05 to about 0.10 equivalent, from about 0.05 to about 0.09 equivalent, from about 0.05 to about 0.08 equivalent, from about 0.05 to about 0.07 equivalent, from about 0.05 to about 0.06 equivalent, from about 0.06 to about 0.15 equivalent, from about 0.06 to about 0.14 equivalent, from about 0.06 to about 0.13 equivalent, from about 0.06 to about 0.12 equivalent, from about 0.06 to about 0.11 equivalent, from about 0.06 to about 0.10 equivalent, from about 0.06 to about 0.09 equivalent, from about 0.06 to about 0.08 equivalent, from about 0.06 to about 0.07 equivalent, from about 0.07 to about 0.15 equivalent, from about 0.07 to about 0.14 equivalent, from about 0.07 to about 0.13 equivalent, from about 0.07 to about 0.12 equivalent, from about 0.07 to about 0.11 equivalent, from about 0.07 to about 0.10 equivalent, from about 0.07 to about 0.09 equivalent, from about 0.07 to about 0.08 equivalent, from about 0.08 to about 0.15 equivalent, from about 0.08 to about 0.14 equivalent, from about 0.08 to about 0.13 equivalent, from about 0.08 to about 0.12 equivalent, from about 0.08 to about 0.11 equivalent, from about 0.08 to about 0.10 equivalent, or from about 0.08 to about 0.09 equivalent of copper salt can be used per equivalent of starting N-(4-chloro-2-iodo-phenyl)-1,3-benzothiazole-6-sulfonamide.

In some embodiments, 5-hexynoic acid is used in an amount from about 1.0 to about 2.0 equivalents, based on the amount of starting N-(4-chloro-2-iodo-phenyl)-1,3-benzothiazole-6-sulfonamide. For example, from about 0.5 to about 2.0 equivalents, from about 0.6 to about 2.0 equivalents, from about 0.7 to about 2.0 equivalents, from about 0.8 to about 2.0 equivalents, from about 0.9 to about 2.0 equivalents, from about 1.0 to about 1.9 equivalents, from about 1.0 to about 1.8 equivalents, from about 1.0 to about 1.7 equivalents, from about 1.0 to about 1.6 equivalents, from about 1.0 to about 1.5 equivalents, from about 1.1 to about 2.0 equivalents, from about 1.1 to about 1.9 equivalents, from about 1.1 to about 1.8 equivalents, from about 1.1 to about 1.7 equivalents, from about 1.1 to about 1.6 equivalents, from about 1.1 to about 1.5 equivalents, from about 1.2 to about 2.0 equivalents, from about 1.2 to about 1.9 equivalents, from about 1.2 to about 1.8 equivalents, from about 1.2 to about 1.7 equivalents, from about 1.2 to about 1.6 equivalents, from about 1.2 to about 1.5 equivalents, from about 1.3 to about 2.0 equivalents, from about 1.3 to about 1.9 equivalents, from about 1.3 to about 1.8 equivalents, from about 1.3 to about 1.7 equivalents, from about 1.3 to about 1.6 equivalents, from about 1.3 to about 1.5 equivalents, from about 1.4 to about 2.0 equivalents, from about 1.4 to about 1.9 equivalents, from about 1.4 to about 1.8 equivalents, from about 1.4 to about 1.7 equivalents, from about 1.4 to about 1.6 equivalents, from about 1.4 to about 1.5 equivalents, from about 1.5 to about 2.0 equivalents, from about 1.5 to about 1.9 equivalents, from about 1.5 to about 1.8 equivalents, from about 1.5 to about 1.7 equivalents, from about 1.5 to about 1.6 equivalents, from about 1.6 to about 2.0 equivalents, from about 1.6 to about 1.9 equivalents, from about 1.6 to about 1.8 equivalents, from about 1.6 to about 1.7 equivalents, from about 1.7 to about 2.0 equivalents, from about 1.7 to about 1.9 equivalents, from about 1.7 to about 1.8 equivalents, from about 1.8 to about 2.0 equivalents, from about 1.8 to about 1.9 equivalents, or from about 1.9 to about 2.0 equivalents of 5-hexynoic acid can be used per equivalent of starting N-(4-chloro-2-iodophenyl)-1,3-benzothiazole-6-sulfonamide.

In some embodiments, the reaction is carried out in a solvent. In some embodiments, the solvent includes, but is not limited to, dichloromethane, chloroform, pyridine, 4-dimethylaminopyridine, N,N-dimethylformamide, dimethyl sulfoxide, acetonitrile, tetrahydrofuran, 2-methyltetrahydrofuran, ethyl acetate, toluene, acetone, methylethylketone (MEK), methylisobutylketone (MIBK), cyclohexanone, methylbutylketone (MBK), water and mixtures thereof. In some embodiments, the solvent is N,N-dimethylformamide, tetrahydrofuran, 2-methyl tetrahydrofuran, water and mixtures thereof.

In some embodiments, the reaction is carried out in the presence of a base. In some embodiments, the base includes, but is not limited to, amines, particularly tertiary amines such as trimethylamine, triethylamine, pyridine, imidazole, 4-dimethylaminopyridine (DMAP), 2,6-lutidine, 1,4-diazabicyclo[2.2.2]octane (DABCO), 1,8-diazabicyclo[5.4.0]undec-7-ene (DBU), 1,5-diazabicyclo[4.3.0]non-5-ene (DBN), or N,N-dimethylphenylamine. In some embodiments, the base is used in an amount of about 1.0 to 20.0 equivalents, based on the amount of starting N-(4-chloro-2-iodo-phenyl)-1,3-benzothiazole-6-sulfonamide. For example, from about 1.0 to about 19.5 equivalents, from about 1.0 to about 19.0 equivalents, from about 1.0 to about 18.5 equivalents, from about 1.0 to about 18.0 equivalents, from about 1.0 to about 17.5 equivalents, from about 1.0 to about 17.0 equivalents, from about 1.0 to about 16.0 equivalents, from about 1.0 to about 16.0 equivalents, from about 1.0 to about 15.5 equivalents, from about 1.0 to about 15.0 equivalents, from about 1.0 to about 14.5 equivalents, from about 1.0 to about 14.0 equivalents, from about 1.0 to about 13.5 equivalents, from about 1.0 to about 13.0 equivalents, from about 1.0 to about 12.5 equivalents, from about 1.0 to about 12.0 equivalents, from about 1.0 to about 11.5 equivalents, from about 1.0 to about 11.0 equivalent, from about 1.0 to about 10.5 equivalent, from about 1.0 to about 10.0 equivalents, from about 1.0 to about 9.5 equivalents, from about 1.0 to about 9.0 equivalents, from about 1.0 to about 8.5 equivalents, from about 1.0 to about 8.0 equivalents, from about 1.0 to about 7.5 equivalents, from about 1.0 to about 7.0 equivalents, from about 1.0 to about 6.5 equivalents, from about 1.0 to about 6.0 equivalents, from about 1.0 to about 5.5 equivalents, from about 1.0 to about 5.0 equivalents, from about 1.0 to about 4.5 equivalents, from about 1.0 to about 4.0 equivalents, from about 1.0 to about 3.5 equivalents, from about 1.0 to about 3.0 equivalents, from about 1.0 to about 2.5 equivalents, from about 1.0 to about 2.0 equivalents, from about 1.0 to about 1.5 equivalents, from about 1.5 to about 20.0 equivalents, from about 1.5 to about 19.5 equivalents, from about 1.5 to about 19.0 equivalents, from about 1.5 to about 18.5 equivalents, from about 1.5 to about 18.0 equivalents, from about 1.5 to about 17.5 equivalents, from about 1.5 to about 17.0 equivalents, from about 1.5 to about 16.0 equivalents, from about 1.5 to about 16.0 equivalents, from about 1.5 to about 15.5 equivalents, from about 1.5 to about 15.0 equivalents, from about 1.5 to about 14.5 equivalents, from about 1.5 to about 14.0 equivalents, from about 1.5 to about 13.5 equivalents, from about 1.5 to about 13.0 equivalents, from about 1.5 to about 12.5 equivalents, from about 1.5 to about 12.0 equivalents, from about 1.5 to about 11.5 equivalents, from about 1.5 to about 11.0 equivalents, from about 1.5 to about 10.5 equivalents, from about 1.5 to about 10.0 equivalents, from about 1.5 to about 9.5 equivalents, from about 1.5 to about 9.0 equivalents, from about 1.5 to about 8.5 equivalents, from about 1.5 to about 8.0 equivalents, from about 1.5 to about 7.5 equivalents, from about 1.5 to about 7.0 equivalents, from about 1.5 to about 6.5 equivalents, from about 1.5 to about 6.0 equivalents, from about 1.5 to about 5.5 equivalents, from about 1.5 to about 5.0 equivalents, from about 1.5 to about 4.5 equivalents, from about 1.5 to about 4.0 equivalents, from about 1.5 to about 3.5 equivalents, from about 1.5 to about 3.0 equivalents, from about 1.5 to about 2.5 equivalents, from about 1.5 to about 2.0 equivalents, from about 2.0 to about 20.0 equivalents, from about 2.0 to about 19.5 equivalents, from about 2.0 to about 19.0 equivalents, from about 2.0 to about 18.5 equivalents, from about 2.0 to about 18.0 equivalents, from about 2.0 to about 17.5 equivalents, from about 2.0 to about 17.0 equivalents, from about 2.0 to about 16.0 equivalents, from about 2.0 to about 16.0 equivalents, from about 2.0 to about 15.5 equivalents, from about 2.0 to about 15.0 equivalents, from about 2.0 to about 14.5 equivalents, from about 2.0 to about 14.0 equivalents, from about 2.0 to about 13.5 equivalents, from about 2.0 to about 13.0 equivalents, from about 2.0 to about 12.5 equivalents, from about 2.0 to about 12.0 equivalents, from about 2.0 to about 11.5 equivalents, from about 2.0 to about 11.0 equivalents, from about 2.0 to about 10.5 equivalents, from about 2.0 to about 10.0 equivalents, from about 2.0 to about 9.5 equivalents, from about 2.0 to about 9.0 equivalents, from about 2.0 to about 8.5 equivalents, from about 2.0 to about 8.0 equivalents, from about 2.0 to about 7.5 equivalents, from about 2.0 to about 7.0 equivalents, from about 2.0 to about 6.5 equivalents, from about 2.0 to about 6.0 equivalents, from about 2.0 to about 5.5 equivalents, from about 2.0 to about 5.0 equivalents, from about 2.0 to about 4.5 equivalents, from about 2.0 to about 4.0 equivalents, from about 2.0 to about 3.5 equivalents, from about 2.0 to about 3.0 equivalents, from about 2.0 to about 2.5 equivalents, from about 2.5 to about 20.0 equivalents, from about 2.5 to about 19.5 equivalents, from about 2.5 to about 19.0 equivalents, from about 2.5 to about 18.5 equivalents, from about 2.5 to about 18.0 equivalents, from about 2.5 to about 17.5 equivalents, from about 2.5 to about 17.0 equivalents, from about 2.5 to about 16.0 equivalents, from about 2.5 to about 16.0 equivalents, from about 2.5 to about 15.5 equivalents, from about 2.5 to about 15.0 equivalents, from about 2.5 to about 14.5 equivalents, from about 2.5 to about 14.0 equivalents, from about 2.5 to about 13.5 equivalents, from about 2.5 to about 13.0 equivalents, from about 2.5 to about 12.5 equivalents, from about 2.5 to about 12.0 equivalents, from about 2.5 to about 11.5 equivalents, from about 2.5 to about 11.0 equivalents, from about 2.5 to about 10.5 equivalents, from about 2.5 to about 10.0 equivalents, from about 2.5 to about 9.5 equivalents, from about 2.5 to about 9.0 equivalents, from about 2.5 to about 8.5 equivalents, from about 2.5 to about 8.0 equivalents, from about 2.5 to about 7.5 equivalents, from about 2.5 to about 7.0 equivalents, from about 2.5 to about 6.5 equivalents, from about 2.5 to about 6.0 equivalents, from about 2.5 to about 5.5 equivalents, from about 2.5 to about 5.0 equivalents, from about 2.5 to about 4.5 equivalents, from about 2.5 to about 4.0 equivalents, from about 2.5 to about 3.5 equivalents, from about 2.5 to about 3.0 equivalents, from about 3.0 to about 20.0 equivalents, from about 3.0 to about 19.5 equivalents, from about 3.0 to about 19.0 equivalents, from about 3.0 to about 18.5 equivalents, from about 3.0 to about 18.0 equivalents, from about 3.0 to about 17.5 equivalents, from about 3.0 to about 17.0 equivalents, from about 3.0 to about 16.0 equivalents, from about 3.0 to about 16.0 equivalents, from about 3.0 to about 15.5 equivalents, from about 3.0 to about 15.0 equivalents, from about 3.0 to about 14.5 equivalents, from about 3.0 to about 14.0 equivalents, from about 3.0 to about 13.5 equivalents, from about 3.0 to about 13.0 equivalents, from about 3.0 to about 12.5 equivalents, from about 3.0 to about 12.0 equivalents, from about 3.0 to about 11.5 equivalents, from about 3.0 to about 11.0 equivalents, from about 3.0 to about 10.5 equivalents, from about 3.0 to about 10.0 equivalents, from about 3.0 to about 9.5 equivalents, from about 3.0 to about 9.0 equivalents, from about 3.0 to about 8.5 equivalents, from about 3.0 to about 8.0 equivalents, from about 3.0 to about 7.5 equivalents, from about 3.0 to about 7.0 equivalents, from about 3.0 to about 6.5 equivalents, from about 3.0 to about 6.0 equivalents, from about 3.0 to about 5.5 equivalents, from about 3.0 to about 5.0 equivalents, from about 3.0 to about 4.5 equivalents, from about 3.0 to about 4.0 equivalents, from about 3.0 to about 3.5 equivalents, from about 3.5 to about 20.0 equivalents, from about 3.5 to about 19.5 equivalents, from about 3.5 to about 19.0 equivalents, from about 3.5 to about 18.5 equivalents, from about 3.5 to about 18.0 equivalents, from about 3.5 to about 17.5 equivalents, from about 3.5 to about 17.0 equivalents, from about 3.5 to about 16.0 equivalents, from about 3.5 to about 16.0 equivalents, from about 3.5 to about 15.5 equivalents, from about 3.5 to about 15.0 equivalents, from about 3.5 to about 14.5 equivalents, from about 3.5 to about 14.0 equivalents, from about 3.5 to about 13.5 equivalents, from about 3.5 to about 13.0 equivalents, from about 3.5 to about 12.5 equivalents, from about 3.5 to about 12.0 equivalents, from about 3.5 to about 11.5 equivalents, from about 3.5 to about 11.0 equivalents, from about 3.5 to about 10.5 equivalents, from about 3.5 to about 10.0 equivalents, from about 3.5 to about 9.5 equivalents, from about 3.5 to about 9.0 equivalents, from about 3.5 to about 8.5 equivalents, from about 3.5 to about 8.0 equivalents, from about 3.5 to about 7.5 equivalents, from about 3.5 to about 7.0 equivalents, from about 3.5 to about 6.5 equivalents, from about 3.5 to about 6.0 equivalents, from about 3.5 to about 5.5 equivalents, from about 3.5 to about 5.0 equivalents, from about 3.5 to about 4.5 equivalents, from about 3.5 to about 4.0 equivalents, from about 4.0 to about 20.0 equivalents, from about 4.0 to about 19.5 equivalents, from about 4.0 to about 19.0 equivalents, from about 4.0 to about 18.5 equivalents, from about 4.0 to about 18.0 equivalents, from about 4.0 to about 17.5 equivalents, from about 4.0 to about 17.0 equivalents, from about 4.0 to about 16.0 equivalents, from about 4.0 to about 16.0 equivalents, from about 4.0 to about 15.5 equivalents, from about 4.0 to about 15.0 equivalents, from about 4.0 to about 14.5 equivalents, from about 4.0 to about 14.0 equivalents, from about 4.0 to about 13.5 equivalents, from about 4.0 to about 13.0 equivalents, from about 4.0 to about 12.5 equivalents, from about 4.0 to about 12.0 equivalents, from about 4.0 to about 11.5 equivalents, from about 4.0 to about 11.0 equivalents, from about 4.0 to about 10.5 equivalents, from about 4.0 to about 10.0 equivalents, from about 4.0 to about 9.5 equivalents, from about 4.0 to about 9.0 equivalents, from about 4.0 to about 8.5 equivalents, from about 4.0 to about 8.0 equivalents, from about 4.0 to about 7.5 equivalents, from about 4.0 to about 7.0 equivalents, from about 4.0 to about 6.5 equivalents, from about 4.0 to about 6.0 equivalents, from about 4.0 to about 5.5 equivalents, from about 4.0 to about 5.0 equivalents, from about 4.0 to about 4.5 equivalents, from about 4.5 to about 20.0 equivalents, from about 4.5 to about 19.5 equivalents, from about 4.5 to about 19.0 equivalents, from about 4.5 to about 18.5 equivalents, from about 4.5 to about 18.0 equivalents, from about 4.5 to about 17.5 equivalents, from about 4.5 to about 17.0 equivalents, from about 4.5 to about 16.0 equivalents, from about 4.5 to about 16.0 equivalents, from about 4.5 to about 15.5 equivalents, from about 4.5 to about 15.0 equivalents, from about 4.5 to about 14.5 equivalents, from about 4.5 to about 14.0 equivalents, from about 4.5 to about 13.5 equivalents, from about 4.5 to about 13.0 equivalents, from about 4.5 to about 12.5 equivalents, from about 4.5 to about 12.0 equivalents, from about 4.5 to about 11.5 equivalents, from about 4.5 to about 11.0 equivalents, from about 4.5 to about 10.5 equivalents, from about 4.5 to about 10.0 equivalents, from about 4.5 to about 9.5 equivalents, from about 4.5 to about 9.0 equivalents, from about 4.5 to about 8.5 equivalents, from about 4.5 to about 8.0 equivalents, from about 4.5 to about 7.5 equivalents, from about 4.5 to about 7.0 equivalents, from about 4.5 to about 6.5 equivalents, from about 4.5 to about 6.0 equivalents, from about 4.5 to about 5.5 equivalents, from about 4.5 to about 5.0 equivalents, from about 5.0 to about 20.0 equivalents, from about 5.0 to about 19.5 equivalents, from about 5.0 to about 19.0 equivalents, from about 5.0 to about 18.5 equivalents, from about 5.0 to about 18.0 equivalents, from about 5.0 to about 17.5 equivalents, from about 5.0 to about 17.0 equivalents, from about 5.0 to about 16.0 equivalents, from about 5.0 to about 16.0 equivalents, from about 5.0 to about 15.5 equivalents, from about 5.0 to about 15.0 equivalents, from about 5.0 to about 14.5 equivalents, from about 5.0 to about 14.0 equivalents, from about 5.0 to about 13.5 equivalents, from about 5.0 to about 13.0 equivalents, from about 5.0 to about 12.5 equivalents, from about 5.0 to about 12.0 equivalents, from about 5.0 to about 11.5 equivalents, from about 5.0 to about 11.0 equivalents, from about 5.0 to about 10.5 equivalents, from about 5.0 to about 10.0 equivalents, from about 5.0 to about 9.5 equivalents, from about 5.0 to about 9.0 equivalents, from about 5.0 to about 8.5 equivalents, from about 5.0 to about 8.0 equivalents, from about 5.0 to about 7.5 equivalents, from about 5.0 to about 7.0 equivalents, from about 5.0 to about 6.5 equivalents, from about 5.0 to about 6.0 equivalents, from about 5.0 to about 5.5 equivalents, from about 5.5 to about 20.0 equivalents, from about 6.0 to about 20.0 equivalents, from about 6.5 to about 20.0 equivalents, from about 7.0 to about 20.0 equivalents, from about 7.5 to about 20.0 equivalents, from about 8.0 to about 20.0 equivalents, from about 8.5 to about 20.0 equivalents, from about 9.0 to about 20.0 equivalents, from about 9.5 to about 20.0 equivalents, from about 10.0 to about 20.0 equivalents, from about 10.5 to about 20.0 equivalents, from about 11.0 to about 20.0 equivalents, from about 11.5 to about 20.0 equivalents, from about 12.0 to about 20.0 equivalents, from about 12.5 to about 20.0 equivalents, from about 13.0 to about 20.0 equivalents, from about 13.5 to about 20.0 equivalents, from about 14.0 to about 20.0 equivalents, from about 14.5 to about 20.0 equivalents, from about 15.0 to about 20.0 equivalents, from about 15.5 to about 20.0 equivalents, from about 16.0 to about 20.0 equivalents, from about 16.5 to about 20.0 equivalents, from about 17.0 to about 20.0 equivalents, from about 17.5 to about 20.0 equivalents, from about 18.0 to about 20.0 equivalents, from about 18.5 to about 20.0 equivalents, from about 19.0 to about 20.0 equivalents based on the amount of starting N-(4-chloro-2-iodo-phenyl)-1,3-benzothiazole-6-sulfonamide. In some embodiment, the base is used in an amount of about 20 equivalents or less, such as about 19 equivalents or less, or else about 18 equivalents or less, 17 equivalents or less, such as about 16 equivalents or less, or else about 15 equivalents or less, 14 equivalents or less, such as about 13 equivalents or less, or else about 12 equivalents or less, 11 equivalents or less, such as equivalents 10 equivalent or less, or else about 9 equivalents or less, 8 equivalents or less, such as about 7 equivalents or less, or else about equivalents or less, 5 equivalents or less, such as about 4 equivalents or less, such as about 3 equivalents or less, or else about 2 equivalents or less, per equivalent of starting N-(4-chloro-2-iodo-phenyl)-1,3-benzothiazole-6-sulfonamide.

In some embodiments, the reaction is carried out at a temperature from about 35°C to about 200°C. For example, the reaction is carried out at a temperature from about 40°C to about 190°C, from about 45°C to about 180°C, from about 50°C to 170°C, from about 55°C to 160°C, from about 60°C to 150°C, from about 65°C to 140°C, from about 70°C to 130°C, from about 75°C to 120°C, from about 80°C to 110°C, from about 85°C to 100°C, from about 90°C to 100°C, from about 40°C to about 100°C, from about 45°C to about 95°C, from about 50°C to 90°C, from about 55°C to 85°C, from about 40°C to 80°C, from about 45°C to 80°C, from about 50°C to 80°C, from about 55°C to 80°C, from about 60°C to 80°C, from about 65°C to 80°C, from about 70°C to 80°C, from about 75°C to about 80°C, from about 40°C to 60°C, from about 45°C to about 60°C, from about 50°C to 60°C, from about 60°C to 100°C, from about 65°C to 100°C, from about 70°C to 100°C, from about 75°C to 100°C, from about 80°C to 100°C, from about 85°C to 100°C, from about 85°C to 110°C, from about 35°C to 80°C.

The inventors have surprisingly found that the use of heterogeneous catalysis with palladium-on-carbon catalyst improves the conversion rate and the selectivity of the manufacturing process. Indeed, the use of a heterogeneous catalyst facilitates easy and complete separation of the catalyst from the reaction product. Moreover, the use of heterogeneous catalysis with palladium-on-carbon catalyst allows obtaining high yield and purity of lanifibranor with the above-mentioned solvents. The recyclability of the catalyst is also improved when using heterogeneous catalysis with palladium-on-carbon catalyst. The use of a heterogeneous catalyst allows reducing the time for completion of the reaction (2 - 7 hours). The use of heterogeneous catalysis renders the present process more economical and more environmentally friendly by using a recyclable catalyst. Indeed, the reaction of N-(4-chloro-2-iodo-phenyl)-1,3-benzothiazole-6-sulfonamide with 5-hexynoic acid yields lanifibranor with a purity, as determined by HPLC (high performance liquid chromatography) analysis, which is ≥ 95%, preferably ≥ 97%, more preferably ≥ 98%, even more preferably ≥ 99%. Lanifibranor is obtained in high yield, i.e. a yield of at least 50%, such as at least 51%, at least 52% at least 53%, at least 54%, at least 55%, at least 56%, at least 57%, at least 58%, at least 59%, at least 60%, at least 61%, at least 62% at least 63%, at least 64%, at least 65%, at least 66%, at least 67%, at least 68%, at least 69%, at least 70%, at least 71%, at least 72% at least 73%, at least 74%, at least 75%, at least 76%, at least 77%, at least 78%, at least 79%, at least 80%, at least 81%, at least 82% at least 83%, at least 84%, at least 85%, at least 86%, at least 87%, at least 88%, at least 89% or at least 90%.

Advantageously, the process for the manufacture of lanifibranor as described above, and comprising reacting N-(4-chloro-2-iodo-phenyl)-1,3-benzothiazole-6-sulfonamide with 5-hexynoic acid in the presence of Pd/C and of a copper salt, can be applied for the manufacture of a deuterated form of lanifibranor.

One of the starting material, 5-hexynoic acid (CAS [53293-00-8]), is commercially available. The other starting material, N-(4-chloro-2-iodo-phenyl)-1,3-benzothiazole-6-sulfonamide, can be obtained via a condensation reaction between 1,3-benzothiazole-6-sulfonyl chloride (CAS [181124-40-3]) and 4-chloro-2-iodoaniline (CAS [63069-48-7]):

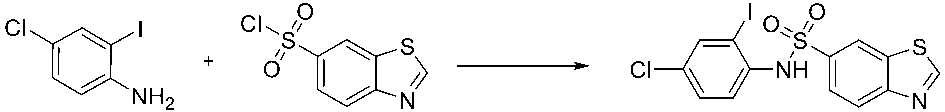

In some embodiments, N-(4-chloro-2-iodo-phenyl)-1,3-benzothiazole-6-sulfonamide can be a deuterated form of N-(4-chloro-2-iodo-phenyl)-1,3-benzothiazole-6-sulfonamide. The deuterated form of N-(4-chloro-2-iodo-phenyl)-1,3-benzothiazole-6-sulfonamide can be obtained according to the method described in the patent application WO2020/021215.

In some embodiments, the condensation reaction is performed in a solvent. In some embodiments, the solvent includes, but is not limited to, dichloromethane, chloroform, pyridine, 4-dimethylaminopyridine, N,N-dimethylformamide, dimethyl sulfoxide, acetonitrile, tetrahydrofuran, ethyl acetate, 2-methyltetrahydrofuran, acetone, methylethylketone (MEK), methylisobutylketone (MIBK), cyclohexanone, methyl butyl ketone (MBK), acetone/water and mixtures thereof. In some embodiments, the solvent is acetonitrile, pyridine, acetone/water, dichloromethane or mixtures thereof.

In some embodiments the condensation reaction is carried out in the presence of a base. In some embodiments, the base includes, but is not limited to, amines, particularly tertiary amines such as trimethylamine, triethylamine, pyridine, imidazole, 4-dimethylaminopyridine, 2,6-lutidine, 1,4-diazabicyclo[2.2.2]octane, 1,8-diazabicyclo[5.4.0]undec-7-ene, 1,5-diazabicyclo[4.3.0]non-5-ene, or N,N-dimethylphenylamine.

In some embodiments, the condensation reaction is carried out at room temperature.

In another aspect, the present disclosure relates to a process for the manufacture of crystalline form beta of lanifibranor, comprising:
a) preparing a hot slurry of lanifibranor in a suitable solvent for lanifibranor,
b) optionally, filtering the hot slurry of lanifibranor,
c) cooling the slurry of lanifibranor to a temperature from about 15°C to about 95°C, and
d) maintaining the slurry of lanifibranor to a temperature from about 15°C to about 95°C, for about 15 minutes to about 72h.

In some embodiments, the hot slurry of lanifibranor of step a) is a hot slurry of polymorphous lanifibranor.

As used herein, polymorphism refers to the phenomenon of multiple crystalline forms of a compound. Within the context of the invention, "polymorphous lanifibranor" encompasses multiple crystalline forms of lanifibranor.

In some embodiments, the suitable solvent used in step a) is selected from, but not limited to, the group consisting of:
- water;
- dimethyl sulfoxide (DMSO);
- alcohol solvents such as methanol, ethanol, 2-nitroethanol, 2-fluoroethanol, 2,2,2-trifluoroethanol, hexafluoroisopropyl alcohol, ethylene glycol, n-propanol (in particular 1-propanol, 2-propanol, isopropyl alcohol), isopropanol, 2-methoxyethanol, 1-butanol, 2-butanol, t-butyl alcohol, 2-ethoxyethanol, diethylene glycol, polyethylene glycol, 1-, 2-, or 3-pentanol, neopentyl alcohol, t-pentyl alcohol, cyclohexanol, phenol, glycerol and the like;
- halogenated hydrocarbons solvents such as dichloromethane, 1,2-dichloroethane, trichloroethylene, perchloroethylene, 1,1,1- trichloroethane, 1,1,2-trichloroethane, chloroform, carbon tetrachloride; ethers such as diethyl ether, diisopropyl ether, methyl t-butyl ether, glyme, diglyme, 1,4-dioxane, 1,3-dioxolane, dibutyl ether, dimethylfuran, 2-methoxyethanol, 2-ethoxyethanol, anisole and the like;
- ether solvents such as methyltetrahydrofuran, tetrahydrofuran glycol monomethyl ether, ethylene glycol monomethyl ether, ethylene glycol dimethyl ether, diethylene glycol monoethyl ether, ethylene glycol dimethyl ether, tetrahydrofuran, 2-methyltetrahydrofuran, monomethyl ether and the like;
- ketone solvents such as acetone, ethyl methyl ketone, diethyl ketone, methyl isobutyl ketone, methyl ethyl ketone, methyl isobutyl ketone, butanone and the like;
- esters solvents such as ethyl acetate, n-propyl acetate, butyl acetate, n-butyl acetate, isopropyl acetate, isobutyl acetate, t-butyl acetate, ethyl formate, methyl acetate, methyl propanoate, ethyl propanoate, methyl butanoate, ethyl butanoate, dimethyl carbonate and the like;
- hydrocarbon solvents such as toluene, xylene, hexane, n-heptane, n-pentane, anisole, ethyl benzene, cyclohexane and the like;
- nitriles solvents such as acetonitrile, propionitrile, butanenitrile, and the like;
- amide solvents such as N,N-dimethylformamide (DMF), N-N-dimethylacetamide, formamide, N-methyl-2-pyrrolidone (NMP) and the like ;
- or a mixture thereof.

In some embodiments, the suitable solvent used in step a) is acetic acid. In some embodiments, the suitable solvent used in step a) is toluene. In some embodiments, the suitable solvent used in step a) is tetrahydrofuran.

In some embodiments, the step a) of preparation of the hot slurry of lanifibranor may be performed optionally in the presence of an antisolvent. In any embodiment of this process, the antisolvent may be selected among the group consisting of a C₁-C₆ alcohol, preferably a C₁-C₄ alcohol, more preferably a C₂-C₄ alcohol, and particularly 1-butanol, water, seawater, salt water (e.g water with NaCl), the list being not limitative.

In some embodiments, the slurry of lanifibranor is heated in step a) to a temperature from about 70°C to about 120°C, such as for example from about 70°C to about 115°C, from about 70°C to about 110°C, from about 75°C to about 105°C, from about 80°C to about 105°C, from about 85°C to about 105°C, from about 90°C to about 105°C, from about 95°C to about 105°C, from about 100°C to 105°C, from about 70°C to about 100°C, from about 75°C to about 100°C, from about 80°C to about 100°C, from about 85°C to about 100°C or from about 90°C to about 100°C.

In some embodiments, step b), when present, includes filtering the hot slurry of lanifibranor. The filtration of step b) can be done by any suitable method. In some embodiments, step b) enables to remove any insoluble material.

In some embodiments, the slurry of lanifibranor is cooled in step c) to a temperature from about 15°C to about 95°C, such as for example from about 16°C to about 95°C, from about 17°C to about 95°C, from about 18°C to about 95°C, from about 19°C to about 95°C, from about 20°C to about 95°C, from about 21°C to about 95°C, from about 22°C to about 95°C, from about 23°C to 95°C, from about 24°C to about 85°C, from about 25°C to 95°C, from about 26°C to about 95°C, from about 27°C to 95°C, from about 28°C to about 95°C, from about 29°C to 95°C, from about 30°C to about 95°C, from about 31°C to 95°C, from about 32°C to about 95°C, from about 33°C to 95°C, from about 34°C to about 95°C, from about 35°C to 95°C, from about 36°C to about 95°C, from about 37°C to 95°C, from about 38°C to about 95°C, from about 39°C to 95°C, from about 40°C to about 95°C, from about 41°C to 95°C, from about 42°C to about 95°C, from about 43°C to 95°C, from about 44°C to about 95°C, from about 45°C to 95°C, from about 46°C to about 95°C, from about 47°C to 95°C, from about 48°C to about 95°C, from about 49°C to 95°C, from about 50°C to about 95°C, from about 51°C to 95°C, from about 52°C to about 95°C, from about 53°C to 95°C, from about 54°C to about 95°C, from about 55°C to 95°C, from about 56°C to about 95°C, from about 57°C to 95°C, from about 58°C to about 95°C, from about 59°C to 95°C, from about 60°C to about 95°C, from about 61°C to 95°C, from about 62°C to about 95°C, from about 63°C to 95°C, from about 64°C to about 95°C, from about 65°C to 95°C, from about 66°C to about 95°C, from about 67°C to 95°C, from about 68°C to about 95°C, from about 69°C to 95°C, from about 70°C to about 95°C, from about 71°C to 95°C, from about 72°C to about 95°C, from about 73°C to 95°C, from about 74°C to about 95°C, from about 75°C to 95°C, from about 76°C to about 95°C, from about 77°C to 95°C, from about 78°C to about 95°C, from about 79°C to 95°C, from about 80°C to about 95°C, from about 81°C to 95°C, from about 82°C to about 95°C, from about 83°C to 95°C, from about 84°C to about 95°C, from about 85°C to about 95°C, from about 86°C to 95°C, from about 87°C to about 95°C, from about 88°C to 95°C, from about 89°C to about 95°C, from about 90°C to about 95°C, from about 91°C to 95°C, from about 92°C to about 95°C, from about 93°C to 95°C, from about 94°C to about 95°C. In some embodiments, the slurry of lanifibranor is cooled in step c) to a temperature from about 20°C to about 85°C, such as for example from about 21°C to about 85°C, from about 22°C to about 85°C, from about 23°C to 85°C, from about 24°C to about 85°C, from about 25°C to 85°C, from about 26°C to about 85°C, from about 27°C to 85°C, from about 28°C to about 85°C, from about 29°C to 85°C, from about 30°C to about 85°C, from about 31°C to 85°C, from about 32°C to about 85°C, from about 33°C to 85°C, from about 34°C to about 85°C, from about 35°C to 85°C, from about 36°C to about 85°C, from about 37°C to 85°C, from about 38°C to about 85°C, from about 39°C to 85°C, from about 40°C to about 85°C, from about 41°C to 85°C, from about 42°C to about 85°C, from about 43°C to 85°C, from about 44°C to about 85°C, from about 45°C to 85°C, from about 46°C to about 85°C, from about 47°C to 85°C, from about 48°C to about 85°C, from about 49°C to 85°C, from about 50°C to about 85°C, from about 51°C to 85°C, from about 52°C to about 85°C, from about 53°C to 85°C, from about 54°C to about 85°C, from about 55°C to 85°C, from about 56°C to about 85°C, from about 57°C to 85°C, from about 58°C to about 85°C, from about 59°C to 85°C, from about 60°C to about 85°C, from about 61°C to 85°C, from about 62°C to about 85°C, from about 63°C to 85°C, from about 64°C to about 85°C, from about 65°C to 85°C, from about 66°C to about 85°C, from about 67°C to 85°C, from about 68°C to about 85°C, from about 69°C to 85°C, from about 70°C to about 85°C, from about 71°C to 85°C, from about 72°C to about 85°C, from about 73°C to 85°C, from about 74°C to about 85°C, from about 75°C to 85°C, from about 76°C to about 85°C, from about 77°C to 85°C, from about 78°C to about 85°C, from about 79°C to 85°C, from about 80°C to about 85°C, from about 81°C to 85°C, from about 82°C to about 85°C, from about 83°C to 85°C, from about 84°C to about 85°C, from about 20°C to 80°C, from about 25°C to 80°C, from about 30°C to 80°C, from about 35°C to 80°C, from about 40°C to 80°C, from about 45°C to 80°C, from about 50°C to about 80°C, from about 55°C to about 80°C, from about 60°C to 80°C, from about 65°C to 80°C, from about 70°C to 80°C. In some embodiments, the slurry of lanifibranor is cooled in step c) to a temperature from about 45°C to about 80°C, such as for example from about 46°C to about 80°C, from about 47°C to 80°C, from about 48°C to about 80°C, from about 49°C to 80°C, from about 50°C to about 80°C, from about 51°C to 80°C, from about 52°C to about 80°C, from about 53°C to 80°C, from about 54°C to about 80°C, from about 55°C to 80°C, from about 56°C to about 80°C, from about 57°C to 80°C, from about 58°C to about 80°C, from about 59°C to 80°C, from about 60°C to about 80°C, from about 61°C to 80°C, from about 62°C to about 80°C, from about 63°C to 80°C, from about 64°C to about 80°C, from about 65°C to 80°C, from about 66°C to about 80°C, from about 67°C to 80°C, from about 68°C to about 80°C, from about 69°C to 80°C, from about 70°C to about 80°C, from about 71°C to 80°C, from about 72°C to about 80°C, from about 73°C to 80°C, from about 74°C to about 80°C, from about 75°C to 80°C, from about 76°C to about 80°C, from about 77°C to 80°C, from about 78°C to about 80°C, from about 79°C to 80°C. In some embodiments, the slurry of lanifibranor is cooled in step c) to a temperature from about 15°C to about 60°C, such as for example from about 16°C to about 60°C, from about 17°C to about 60°C, from about 18°C to about 60°C, from about 19°C to about 60°C, from about 20°C to about 60°C, from about 21°C to about 60°C, from about 22°C to about 60°C, from about 23°C to 60°C, from about 24°C to about 85°C, from about 25°C to 60°C, from about 26°C to about 60°C, from about 27°C to 60°C, from about 28°C to about 60°C, from about 29°C to 60°C, from about 30°C to about 60°C, from about 31°C to 60°C, from about 32°C to about 60°C, from about 33°C to 60°C, from about 34°C to about 60°C, from about 35°C to 60°C, from about 36°C to about 60°C, from about 37°C to 60°C, from about 38°C to about 60°C, from about 39°C to 60°C, from about 40°C to about 60°C, from about 41°C to 60°C, from about 42°C to about 60°C, from about 43°C to 60°C, from about 44°C to about 60°C, from about 45°C to 60°C, from about 46°C to about 60°C, from about 47°C to 60°C, from about 48°C to about 60°C, from about 49°C to 60°C, from about 50°C to about 60°C, from about 51°C to 60°C, from about 52°C to about 60°C, from about 53°C to 60°C, from about 54°C to about 60°C, from about 55°C to 60°C, from about 56°C to about 60°C, from about 57°C to 60°C, from about 58°C to about 60°C, from about 59°C to 60°C. In some embodiments, the slurry of lanifibranor is cooled in step c) to a temperature from about 15°C to about 45°C, such as for example from about 16°C to about 44°C, from about 17°C to about 43°C, from about 18°C to about 42°C, from about 19°C to about 41°C, from about 20°C to about 40°C, from about 21°C to about 39°C, from about 22°C to about 38°C, from about 23°C to 37°C, from about 24°C to about 36°C, from about 25°C to 35°C, from about 26°C to about 34°C, from about 27°C to 33°C, from about 28°C to about 32°C, from about 29°C to 31°C. In some embodiments, the slurry of lanifibranor is cooled in step c) to a temperature from about 15°C to about 45°C, such as for example from about 16°C to about 45°C, from about 17°C to about 45°C, from about 18°C to about 45°C, from about 19°C to about 45°C, from about 20°C to about 45°C, from about 21°C to about 45°C, from about 22°C to about 45°C, from about 23°C to 45°C, from about 24°C to about 85°C, from about 25°C to 45°C, from about 26°C to about 45°C, from about 27°C to 45°C, from about 28°C to about 45°C, from about 29°C to 45°C, from about 30°C to about 45°C, from about 31°C to 45°C, from about 32°C to about 45°C, from about 33°C to 45°C, from about 34°C to about 45°C, from about 35°C to 45°C, from about 36°C to about 45°C, from about 37°C to 45°C, from about 38°C to about 45°C, from about 39°C to 45°C, from about 40°C to about 45°C, from about 41°C to 45°C, from about 42°C to about 45°C, from about 43°C to 45°C, from about 44°C to about 45°C.

In some embodiments, the slurry of lanifibranor is cooled in step c) to a temperature from about 15°C to 95°C, such as for example from about 20°C to 95°C, from about 25°C to 95°C, from about 30°C to 95°C, from about 35°C to 95°C, from about 40°C to 95°C, from about 45°C to 95°C, from about 50°C to about 95°C, from about 55°C to about 95°C, from about 60°C to 95°C, from about 65°C to 95°C, from about 70°C to 95°C, from about 75°C to 95°C, from about 80°C to 95°C, from about 85°C to 95°C, from about 90°C to 95°C and from about 92°C to 95°C. In some embodiments, the slurry of lanifibranor is cooled in step c) to a temperature of for example 15°C, 16°C, 17°C, 18°C, 19°C, 20°C, 21°C, 22°C, 23°C, 24°C, 25°C, 26°C, 27°C, 28°C, 29°C, 30°C, 31°C, 32°C, 33°C, 34°C, 35°C, 36°C, 37°C, 38°C, 39°C, 40°C, 41°C, 42°C, 43°C, 44°C, 45°C, 46°C, 47°C, 48°C, 49°C, 50°C, 51°C, 52°C, 53°C, 54°C, 55°C, 56°C, 57°C, 58°C, 59°C, 60°C, 61°C, 62°C, 63°C, 64°C, 65°C, 66°C, 67°C, 68°C, 69°C, 70°C, 71°C, 72°C, 73°C, 74°C, 75°C, 76°C, 77°C, 78°C, 79°C, 80°C, 81°C, 82°C, 83°C, 84°C, 85°C, 86°C, 87°C, 88°C, 89°C, 90°C, 91°C, 92°C, 93°C, 94°C, or 95°C.

In some embodiments, the slurry of lanifibranor is cooled in step c) to the above-mentioned temperature over a period of time from about 30 min to about 15 hours, such as for example from about 30 min to about 14 hours, from about 30 min to about 13 hours, from about 30 min to about 12 hours, from about 30 min to about 11 hours, from about 30 min to about 10 hours, from about 30 min to about 9 hours, from about 30 min to about 8 hours, from about 30 min to about 7 hours, from about 30 min to about 6 hours, from about 30 min to about 5 hours, from about 30 min to about 4 hours, from about 30 min to about 3 hours, from about 30 min to about 2 hours, from about 30 min to about 1 hour.

In some embodiments, the slurry of lanifibranor is maintained in step d) at a temperature from about 15°C to about 95°C, such as for example from about 16°C to about 95°C, from about 17°C to about 95°C, from about 18°C to about 95°C, from about 19°C to about 95°C, from about 20°C to about 95°C, from about 21°C to about 95°C, from about 22°C to about 95°C, from about 23°C to 95°C, from about 24°C to about 85°C, from about 25°C to 95°C, from about 26°C to about 95°C, from about 27°C to 95°C, from about 28°C to about 95°C, from about 29°C to 95°C, from about 30°C to about 95°C, from about 31°C to 95°C, from about 32°C to about 95°C, from about 33°C to 95°C, from about 34°C to about 95°C, from about 35°C to 95°C, from about 36°C to about 95°C, from about 37°C to 95°C, from about 38°C to about 95°C, from about 39°C to 95°C, from about 40°C to about 95°C, from about 41°C to 95°C, from about 42°C to about 95°C, from about 43°C to 95°C, from about 44°C to about 95°C, from about 45°C to 95°C, from about 46°C to about 95°C, from about 47°C to 95°C, from about 48°C to about 95°C, from about 49°C to 95°C, from about 50°C to about 95°C, from about 51°C to 95°C, from about 52°C to about 95°C, from about 53°C to 95°C, from about 54°C to about 95°C, from about 55°C to 95°C, from about 56°C to about 95°C, from about 57°C to 95°C, from about 58°C to about 95°C, from about 59°C to 95°C, from about 60°C to about 95°C, from about 61°C to 95°C, from about 62°C to about 95°C, from about 63°C to 95°C, from about 64°C to about 95°C, from about 65°C to 95°C, from about 66°C to about 95°C, from about 67°C to 95°C, from about 68°C to about 95°C, from about 69°C to 95°C, from about 70°C to about 95°C, from about 71°C to 95°C, from about 72°C to about 95°C, from about 73°C to 95°C, from about 74°C to about 95°C, from about 75°C to 95°C, from about 76°C to about 95°C, from about 77°C to 95°C, from about 78°C to about 95°C, from about 79°C to 95°C, from about 80°C to about 95°C, from about 81°C to 95°C, from about 82°C to about 95°C, from about 83°C to 95°C, from about 84°C to about 95°C, from about 85°C to about 95°C, from about 86°C to 95°C, from about 87°C to about 95°C, from about 88°C to 95°C, from about 89°C to about 95°C, from about 90°C to about 95°C, from about 91°C to 95°C, from about 92°C to about 95°C, from about 93°C to 95°C, from about 94°C to about 95°C. In some embodiments, the slurry of lanifibranor is maintained in step d) at a temperature from about 20°c to about 85°C, for example from about 21°C to about 85°C, from about 22°C to about 85°C, from about 23°C to 85°C, from about 24°C to about 85°C, from about 25°C to 85°C, from about 26°C to about 85°C, from about 27°C to 85°C, from about 28°C to about 85°C, from about 29°C to 85°C, from about 30°C to about 85°C, from about 31°C to 85°C, from about 32°C to about 85°C, from about 33°C to 85°C, from about 34°C to about 85°C, from about 35°C to 85°C, from about 36°C to about 85°C, from about 37°C to 85°C, from about 38°C to about 85°C, from about 39°C to 85°C, from about 40°C to about 85°C, from about 41°C to 85°C, from about 42°C to about 85°C, from about 43°C to 85°C, from about 44°C to about 85°C, from about 45°C to 85°C, from about 46°C to about 85°C, from about 47°C to 85°C, from about 48°C to about 85°C, from about 49°C to 85°C, from about 50°C to about 85°C, from about 51°C to 85°C, from about 52°C to about 85°C, from about 53°C to 85°C, from about 54°C to about 85°C, from about 55°C to 85°C, from about 56°C to about 85°C, from about 57°C to 85°C, from about 58°C to about 85°C, from about 59°C to 85°C, from about 60°C to about 85°C, from about 61°C to 85°C, from about 62°C to about 85°C, from about 63°C to 85°C, from about 64°C to about 85°C, from about 65°C to 85°C, from about 66°C to about 85°C, from about 67°C to 85°C, from about 68°C to about 85°C, from about 69°C to 85°C, from about 70°C to about 85°C, from about 71°C to 85°C, from about 72°C to about 85°C, from about 73°C to 85°C, from about 74°C to about 85°C, from about 75°C to 85°C, from about 76°C to about 85°C, from about 77°C to 85°C, from about 78°C to about 85°C, from about 79°C to 85°C, from about 80°C to about 85°C, from about 81°C to 85°C, from about 82°C to about 85°C, from about 83°C to 85°C, from about 84°C to about 85°C. In some embodiments, the slurry of lanifibranor is maintained in step d) at a temperature from about 45°C to about 80°C, such as for example from about 46°C to about 80°C, from about 47°C to 80°C, from about 48°C to about 80°C, from about 49°C to 80°C, from about 50°C to about 80°C, from about 51°C to 80°C, from about 52°C to about 80°C, from about 53°C to 80°C, from about 54°C to about 80°C, from about 55°C to 80°C, from about 56°C to about 80°C, from about 57°C to 80°C, from about 58°C to about 80°C, from about 59°C to 80°C, from about 60°C to about 80°C, from about 61°C to 80°C, from about 62°C to about 80°C, from about 63°C to 80°C, from about 64°C to about 80°C, from about 65°C to 80°C, from about 66°C to about 80°C, from about 67°C to 80°C, from about 68°C to about 80°C, from about 69°C to 80°C, from about 70°C to about 80°C, from about 71°C to 80°C, from about 72°C to about 80°C, from about 73°C to 80°C, from about 74°C to about 80°C, from about 75°C to 80°C, from about 76°C to about 80°C, from about 77°C to 80°C, from about 78°C to about 80°C, from about 79°C to 80°C. In some embodiments, the slurry of lanifibranor is maintained in step d) at a temperature from about 15°C to about 60°C, such as for example from about 16°C to about 60°C, from about 17°C to about 60°C, from about 18°C to about 60°C, from about 19°C to about 60°C, from about 20°C to about 60°C, from about 21°C to about 60°C, from about 22°C to about 60°C, from about 23°C to 60°C, from about 24°C to about 85°C, from about 25°C to 60°C, from about 26°C to about 60°C, from about 27°C to 60°C, from about 28°C to about 60°C, from about 29°C to 60°C, from about 30°C to about 60°C, from about 31°C to 60°C, from about 32°C to about 60°C, from about 33°C to 60°C, from about 34°C to about 60°C, from about 35°C to 60°C, from about 36°C to about 60°C, from about 37°C to 60°C, from about 38°C to about 60°C, from about 39°C to 60°C, from about 40°C to about 60°C, from about 41°C to 60°C, from about 42°C to about 60°C, from about 43°C to 60°C, from about 44°C to about 60°C, from about 45°C to 60°C, from about 46°C to about 60°C, from about 47°C to 60°C, from about 48°C to about 60°C, from about 49°C to 60°C, from about 50°C to about 60°C, from about 51°C to 60°C, from about 52°C to about 60°C, from about 53°C to 60°C, from about 54°C to about 60°C, from about 55°C to 60°C, from about 56°C to about 60°C, from about 57°C to 60°C, from about 58°C to about 60°C, from about 59°C to 60°C. In some embodiments, the slurry of lanifibranor is maintained in step d) at a temperature from about 15°C to about 45°C, such as for example from about 16°C to about 44°C, from about 17°C to about 43°C, from about 18°C to about 42°C, from about 19°C to about 41°C, from about 20°C to about 40°C, from about 21°C to about 39°C, from about 22°C to about 38°C, from about 23°C to 37°C, from about 24°C to about 36°C, from about 25°C to 35°C, from about 26°C to about 34°C, from about 27°C to 33°C, from about 28°C to about 32°C, from about 29°C to 31°C. In some embodiments, the slurry of lanifibranor is maintained in step d) at a temperature from about 15°C to about 45°C, such as for example from about 16°C to about 45°C, from about 17°C to about 45°C, from about 18°C to about 45°C, from about 19°C to about 45°C, from about 20°C to about 45°C, from about 21°C to about 45°C, from about 22°C to about 45°C, from about 23°C to 45°C, from about 24°C to about 85°C, from about 25°C to 45°C, from about 26°C to about 45°C, from about 27°C to 45°C, from about 28°C to about 45°C, from about 29°C to 45°C, from about 30°C to about 45°C, from about 31°C to 45°C, from about 32°C to about 45°C, from about 33°C to 45°C, from about 34°C to about 45°C, from about 35°C to 45°C, from about 36°C to about 45°C, from about 37°C to 45°C, from about 38°C to about 45°C, from about 39°C to 45°C, from about 40°C to about 45°C, from about 41°C to 45°C, from about 42°C to about 45°C, from about 43°C to 45°C, from about 44°C to about 45°C.

In some embodiments, the slurry of lanifibranor is maintained in step d) at a temperature from about 15°C to 95°C, such as for example from about 20°C to 95°C, from about 25°C to 95°C, from about 30°C to 95°C, from about 35°C to 95°C, from about 40°C to 95°C, from about 45°C to 95°C, from about 50°C to about 95°C, from about 55°C to about 95°C, from about 60°C to 95°C, from about 65°C to 95°C, from about 70°C to 95°C, from about 75°C to 95°C, from about 80°C to 95°C, from about 85°C to 95°C, from about 90°C to 95°C and from about 92°C to 95°C. In some embodiments, the slurry of lanifibranor is maintained in step d) at a temperature of for example 15°C, 16°C, 17°C, 18°C, 19°C, 20°C, 21°C, 22°C, 23°C, 24°C, 25°C, 26°C, 27°C, 28°C, 29°C, 30°C, 31°C, 32°C, 33°C, 34°C, 35°C, 36°C, 37°C, 38°C, 39°C, 40°C, 41°C, 42°C, 43°C, 44°C, 45°C, 46°C, 47°C, 48°C, 49°C, 50°C, 51°C, 52°C, 53°C, 54°C, 55°C, 56°C, 57°C, 58°C, 59°C, 60°C, 61°C, 62°C, 63°C, 64°C, 65°C, 66°C, 67°C, 68°C, 69°C, 70°C, 71°C, 72°C, 73°C, 74°C, 75°C, 76°C, 77°C, 78°C, 79°C, 80°C, 81°C, 82°C, 83°C, 84°C, 85°C, 86°C, 87°C, 88°C, 89°C, 90°C, 91°C, 92°C, 93°C, 94°C, or 95°C.

In some embodiments, step d) is carried out for a period of time of about 15 minutes to about 72 hours, such as for example about 30 minutes to about 72 hours, about 45 minutes to 72 hours, about 1 hour to 72 hours, about 2 hours to 72 hours, about 3 hours to 72 hours, about 4 hours to 72 hours, about 15 minutes to about 48 hours, about 30 minutes to about 48 hours, about 45 minutes to 48 hours, about 1 hour to 48 hours, about 2 hours to 48 hours, about 3 hours to 48 hours, about 4 hours to 48 hours, about 5 hours to about 48 hours, about 1 hour to 30 hours, about 5 hours to about 30 hours, about 10 hours to about 30 hours, or about 15 hours to about 25 hours, about 15 minutes to about 25 hours, about 30 minutes to about 25 hours, about 45 minutes to 25 hours, about 1 hour to 25 hours, about 2 hours to 25 hours, about 3 hours to 25 hours, about 4 hours to 25 hours, about 5 hours to 25 hours, about 6 hours to 25 hours, about 7 hours to 25 hours, about 8 hours to 25 hours, about 9 hours to 25 hours, about 10 hours to 25 hours, about 11 hours to 25 hours, about 12 hours to 25 hours, about 13 hours to 25 hours, about 14 hours to 25 hours, about 15 hours to 25 hours, about 1 hour to 20 hours, about 2 hours to 20 hours, about 3 hours to 20 hours, about 4 hours to 20 hours, about 5 hours to 20 hours, about 6 hours to 20 hours, about 7 hours to 20 hours, about 8 hours to 20 hours, about 9 hours to 20 hours, about 10 hours to 20 hours, about 11 hours to 20 hours, about 12 hours to 20 hours, about 13 hours to 20 hours, about 14 hours to 20 hours, about 15 hours to 20 hours, about 1 hour to 15 hours, about 2 hours to 15 hours, about 3 hours to 15 hours, about 4 hours to 15 hours, about 5 hours to 15 hours, about 6 hours to 15 hours, about 7 hours to 15 hours, about 8 hours to 15 hours, about 9 hours to 15 hours, about 10 hours to 15 hours.

In some embodiments, the temperature in step d) is identical to the temperature in step c).

In some embodiments, seeds of crystalline form beta of lanifibranor are added during step c) and/or during step d). In some embodiments, seeds of crystalline form beta of lanifibranor are added during step c). In some embodiments, seeds of crystalline form beta of lanifibranor are added during step d). In some embodiments, seeds of crystalline form beta of lanifibranor are added during step c) and step d). In some embodiments, no seeds of crystalline form beta of lanifibranor are added during the reaction.

In some embodiments, steps c) and d) are repeated from one to 10 times, such as for example once, twice, three times, four times, or five times. In some embodiments, steps c) and d) are repeated from one to 10 times, such as for example once, twice, three times, four times, or five times, with different ranges of temperatures, and in particular with decreasing temperatures. For example, if steps c) and d) are repeated twice, the slurry of lanifibranor can be first cooled in step c) to a temperature from about 85°C to about 95°C, and maintained in step d) at a temperature from about 85°C to about 95°C, and then again cooled in step c) to a temperature from about 35°C to about 85°C, and maintained in step d) at a temperature from about 35°C to about 85°C. For example, if steps c) and d) are repeated three times, the slurry of lanifibranor can be first cooled in step c) to a temperature from about 85°C to about 95°C, and maintained in step d) at a temperature from about 85°C to about 95°C, then again cooled in step c) to a temperature from about 45°C to about 85°C, and maintained in step d) at a temperature from about 45°C to about 85°C, and finally again cooled in step c) to a temperature from about 15°C to about 45°C, and maintained in step d) at a temperature from about 15°C to about 45°C. For example, if steps c) and d) are repeated four times, the slurry of lanifibranor can be first cooled in step c) to a temperature from about 85°C to about 95°C, and maintained in step d) at a temperature from about 85°C to about 95°C, then again cooled in step c) to a temperature from about 60°C to about 85°C, and maintained in step d) at a temperature from about 60°C to about 85°C, then again cooled in step c) to a temperature from about 45°C to about 60°C, and maintained in step d) at a temperature from about 45°C to about 65°C, and finally again cooled in step c) to a temperature from about 15°C to about 45°C, and maintained in step d) at a temperature from about 15°C to about 45°C.

The process described above yields crystalline form beta of lanifibranor, which is recovered by methods well-known in the art such as allowing the slurry to return to room temperature, filtering the slurry and drying the solid product filtered.

In one aspect, the present disclosure relates to a process for the manufacture of lanifibranor, said process comprising:
a) reacting N-(4-chloro-2-iodo-phenyl)-1,3-benzothiazole-6-sulfonamide with 5-hexynoic acid in the presence of Pd/C and of a copper salt,
b) recovering lanifibranor.

In one aspect, the present disclosure relates to a process for the manufacture of a deuterated form of lanifibranor, said process comprising:
a) reacting a deuterated form of N-(4-chloro-2-iodo-phenyl)-1,3-benzothiazole-6-sulfonamide with 5-hexynoic acid in the presence of Pd/C and of a copper salt,
b) recovering the deuterated form of lanifibranor.

In one aspect, the present disclosure relates to a process for the manufacture of crystalline form beta of lanifibranor, said process comprising:
a) reacting N-(4-chloro-2-iodo-phenyl)-1,3-benzothiazole-6-sulfonamide with 5-hexynoic acid in the presence of Pd/C and of a copper salt,
b) recovering lanifibranor,
c) preparing a hot slurry of lanifibranor in a suitable solvent for lanifibranor;
d) optionally, filtering the hot slurry of lanifibranor;
e) cooling the slurry of lanifibranor to a temperature from about 15°C to about 95°C; and
f) maintaining the slurry of lanifibranor to a temperature from about 15°C to about 95°C for about 15 minutes to about 72h.

Crystalline form beta of lanifibranor, as obtained by the process described above, displays flowability and compressibility properties which are compatible for subsequent processing into a pharmaceutical form. In particular, crystalline form beta of lanifibranor has an angle of repose comprised between about 31° and about 37°, which corresponds to a good/fair flowability according to European Pharmacopoeia 11.0, paragraph 2.9.16. "Flowability". In some embodiments, the angle of repose is comprised between 32° and 37°, advantageously between 33° and 36°, advantageously between 33° and 35°, advantageously between 33° and 34°. In some embodiments, the angle of repose is 37°, advantageously 36°, advantageously 35°, advantageously 34°, advantageously 33°, advantageously 32°, advantageously 31°.

In some embodiments, crystalline form beta of lanifibranor has a compressibility index of about 11 to about 15 and a Hausner ratio of about 1.12 to about 1.18, which corresponds to a good flowability according to European Pharmacopoeia 11.0, paragraph 2.9.34. "Bulk density and tapped density of powders" - "Measure of powder compressibility".

In some embodiments, crystalline form beta of lanifibranor of the invention has a compressibility index of about 11 to about 15, such as for example of about 12 to about 14, or of about 12 to about 13. In some embodiments, crystalline form beta of lanifibranor has a Hausner ratio of about 1.12 to about 1.18, such as for example of about 1.13 to about 1.17, or of about 1.14 to about 1.16.

Crystalline form beta of lanifibranor also has a compressibility index of about 16 to about 25 and a Hausner ratio of about 1.19 to about 1.34, which corresponds to a fair/passable flowability according to European Pharmacopoeia 11.0, paragraph 2.9.34. "Bulk density and tapped density of powders" - - "Measure of powder compressibility". In some embodiments, crystalline form beta of lanifibranor of the invention has a compressibility index of about 16 to about 25, such as for example of about 16 to about 22, or of about 18 to about 21. In some embodiments, crystalline form beta of lanifibranor has a Hausner ratio of about 1.19 to about 1.34, such as for example of about 1.19 to about 1.30, or of about 1.22 to about 1.28. In some embodiments, crystalline form beta of lanifibranor has a compressibility index of 20.64 and a Hausner ratio of 1.26.

Crystalline form beta of lanifibranor is characterized by its powder X-ray diffraction (PXRD) pattern. One skilled in the art will appreciate that, with respect to X-ray diffraction peak positions (2θ), said positions will show some variability, typically as much as ±0.2°. Further, one skilled in the art will appreciate that relative peak intensities will show inter-apparatus variability, as well as variability due to the degree of crystallinity, and should be taken as qualitative measures only.

In some embodiments, crystalline form beta of lanifibranor has a PXRD pattern comprising one or more peaks at 2θ values selected from 16.0° ± 0.2°, 18.7° ± 0.2° and 24.6° ± 0.2° 2θ, as measured using an X-ray wavelength of 1.5406 *Å*.

In some embodiments, crystalline form beta of lanifibranor has a PXRD pattern further comprising at least one peak at 2θ values selected from 11.4° ± 0.2°, 18.0 ± 0.2°, 21.2 ± 0.2°, 22.8 ± 0.2°, 23.5 ± 0.2°, 26.1 ±0.2° and 26.7° ± 0.2° 2θ, as measured using an X-ray wavelength of 1.5406 *Å*.

In some embodiments, the PXRD pattern further comprises one or more additional peaks at 2θ values selected from the peaks in Table 1.

**Table 1**

| ***2θ position (°)*** | ***Relative intensity*** | ***2θ position (°)*** | ***Relative intensity*** |
|---|---|---|---|
| 9.3 | 4.6% | 28.2 | 20.5% |
| 10.5 | 2.5% | 28.6 | 3.3% |
| 11.1 | 21.2% | 28.7 | 1.7% |
| 11.4 | 60.4% | 29.0 | 1.0% |
| 12.0 | 40.0% | 29.2 | 3.0% |
| 12.6 | 5.7% | 29.4 | 3.1% |
| 13.2 | 5.3% | 30.0 | 23.2% |
| 13.4 | 2.8% | 30.3 | 9.6% |
| 14.3 | 24.2% | 30.5 | 8.1% |
| 15.0 | 21.4% | 30.7 | 5.5% |
| 16.0 | 100.0% | 31.7 | 4.5% |
| 16.4 | 6.5% | 32.1 | 8.9% |
| 18.0 | 55.4% | 32.3 | 13.6% |
| 18.3 | 41.4% | 32.7 | 5.5% |
| 18.7 | 91.5% | 32.9 | 9.1% |
| 18.9 | 20.0% | 33.2 | 14.0% |
| 19.9 | 39.1% | 33.3 | 9.9% |
| 20.2 | 42.3% | 33.6 | 1.8% |
| 20.6 | 36.8% | 34.4 | 4.3% |
| 21.2 | 47.4% | 34.5 | 3.4% |
| 22.1 | 12.2% | 34.8 | 8.0% |
| 22.2 | 10.4% | 35.0 | 9.6% |
| 22.5 | 7.8% | 35.4 | 1.5% |
| 22.8 | 77.1% | 36.2 | 14.6% |
| 23.3 | 3.9% | 36.3 | 11.2% |
| 23.5 | 50.9% | 36.5 | 1.5% |
| 23.9 | 8.3% | 37.0 | 10.0% |
| 24.0 | 31.2% | 37.2 | 7.3% |
| 24.6 | 91.7% | 38.0 | 1.7% |
| 24.8 | 39.5% | 38.1 | 1.0% |
| 25.1 | 3.8% | 38.4 | 3.9% |
| 25.2 | 7.8% | 38.5 | 8.7% |
| 25.5 | 7.9% | 38.6 | 5.2% |
| 26.1 | 46.6% | 39.1 | 1.5% |
| 26.7 | 64.6% | 39.4 | 1.8% |
| 27.3 | 18.8% | 39.8 | 4.3% |
| 27.7 | 2.2% | | |

In some embodiments, the PXRD pattern of crystalline form beta of lanifibranor is substantially in accordance with figure 1.

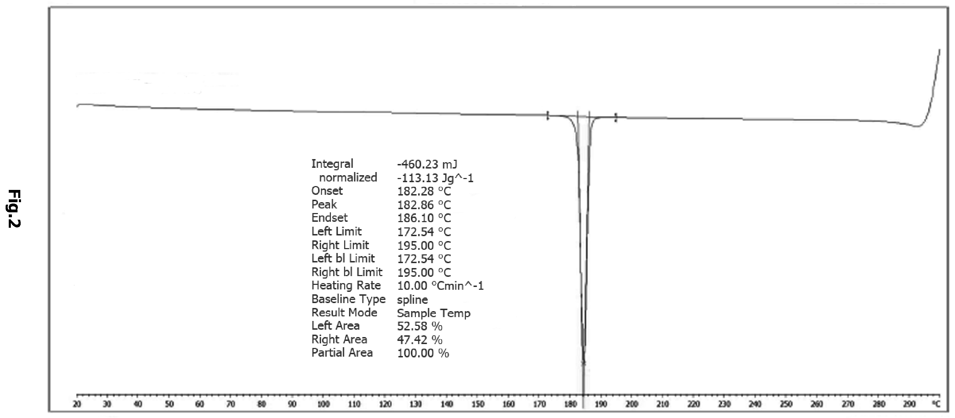

Crystalline form beta of lanifibranor is also characterized by its differential scanning calorimetry (DSC) curve. In some embodiments, crystalline form beta of lanifibranor has a DSC curve comprising an endothermic peak at 182.3°C. In some embodiments, said DSC curve is substantially in accordance with figure 2.

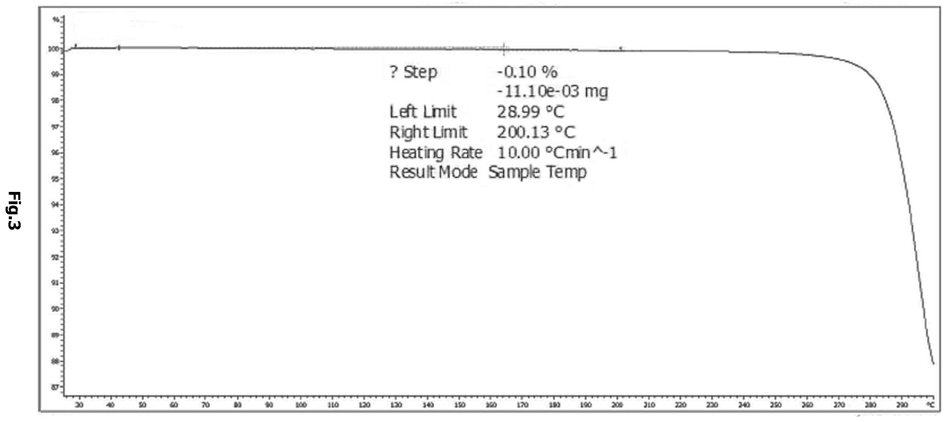

Crystalline form beta of lanifibranor is also characterized by its thermogravimetry (TGA) curve. In some embodiments, the TGA curve of crystalline form beta of lanifibranor does not highlight any significant weight loss over the temperature range 25 - 200°C. Above 250°C, the weight loss observed likely corresponds to degradation. In some embodiments, said TGA curve is substantially in accordance with figure 3.

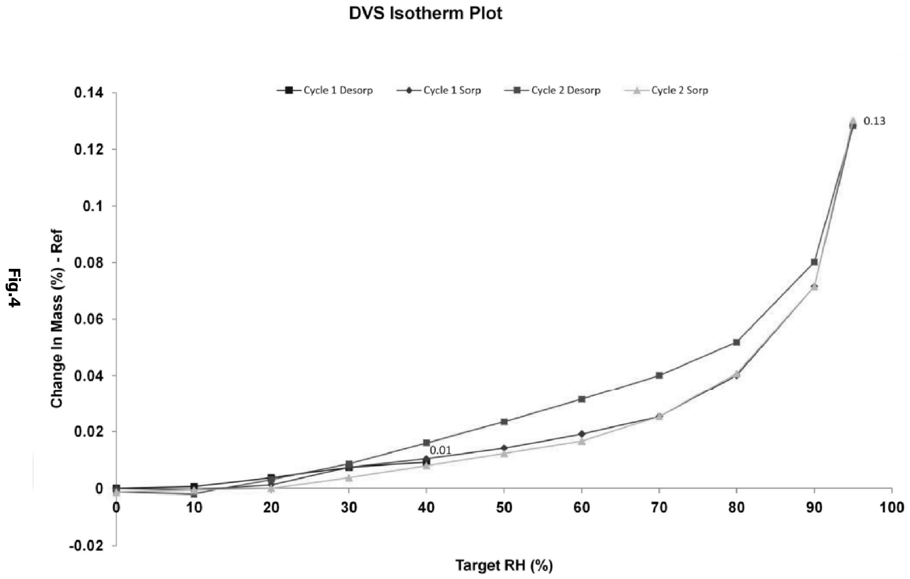

Crystalline form beta of lanifibranor is also characterized by its dynamic vapor sorption (DVS). Analysis performed on said crystalline form does not exhibit significant weight variations over the range of relative humidity values investigated: a maximum uptake of + 0.1% was observed on the range 0% RH - 95% RH. In some embodiments, the DVS isotherm plot of crystalline form beta of lanifibranor is substantially in accordance with figure 4.

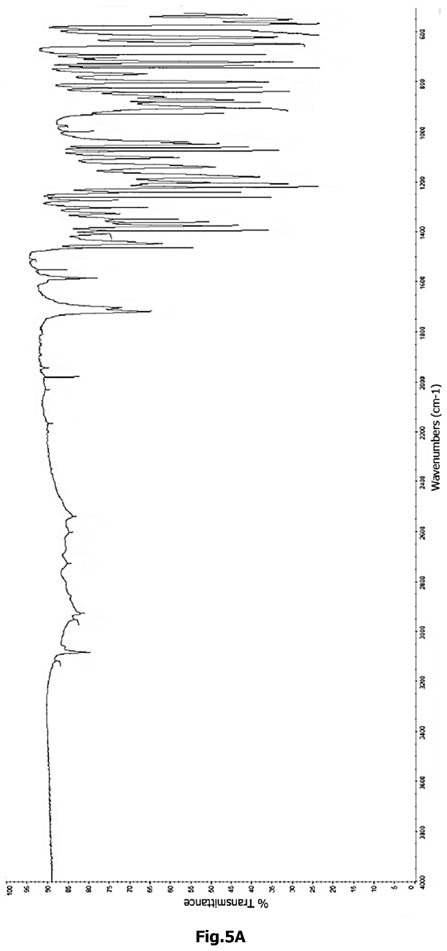

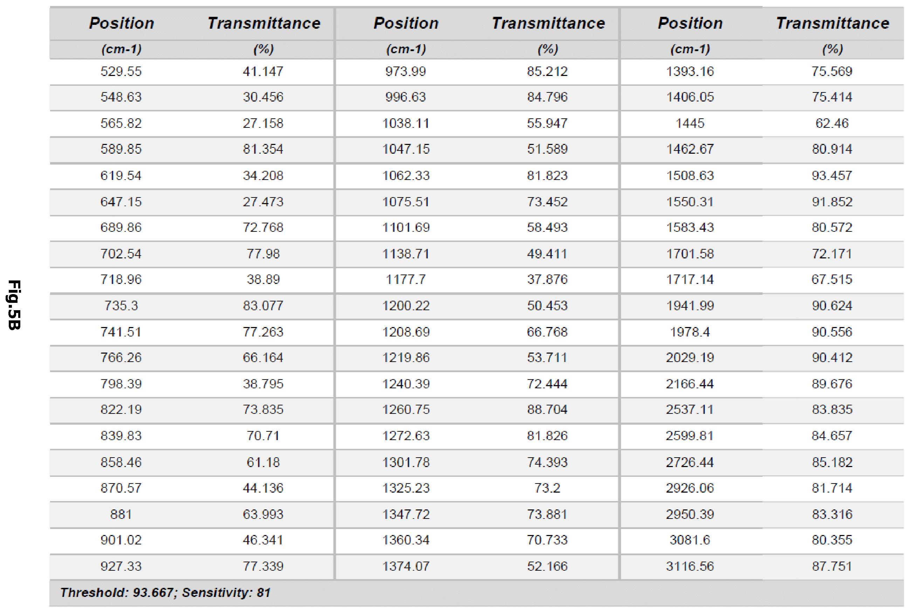

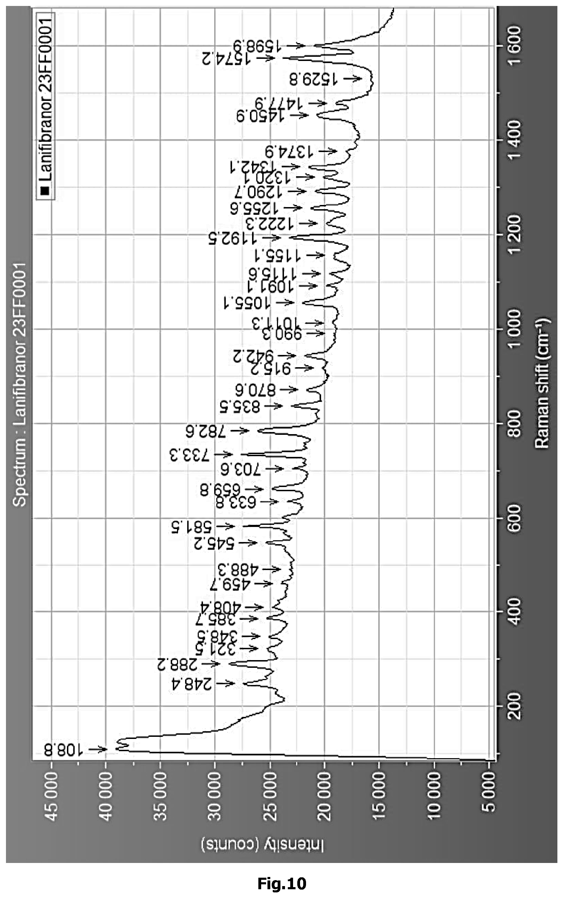

Crystalline form beta of lanifibranor is also characterized by its infrared (IR) spectrum. In some embodiments, said IR spectrum is substantially in accordance with figures 5A and 5B. Crystalline form beta of lanifibranor is also characterized by its Raman spectrum comprising one or more peaks selected from the peaks in Table 2 hereafter. In some embodiments, said Raman spectrum is substantially in accordance with figure 10.

Lanifibranor can be administered in the form of a pharmaceutical composition. Administration in vivo may be by any routes, including oral, parenteral, topical, intramuscular, intranasal, sublingual, intratracheal, inhalation, ocular, vaginal and rectal. Depending on the intended mode of in vivo administration, said composition may be in a solid dosage form, a semi-solid dosage form or a liquid dosage form, the list being not limitative. In some embodiments, the pharmaceutical composition is a solid dosage form. Exemplary solid dosage forms include tablets, capsules, stick-packs, sachets, lozenges, powders, pills, or granules. Preferred solid dosage forms include tablets, capsules and stick-packs, tablets being especially preferred. Advantageously, the compositions are administered in unit dosage forms suitable for single administration of precise dosage amounts. The compositions may also include, depending on the formulation desired, one or more pharmaceutically acceptable excipient(s). The choice of excipient(s) will to a large extent depend on factors such as the particular mode of administration, the effect of the excipient on solubility and stability, and the nature of the dosage form. Pharmaceutical compositions of the invention can be prepared by conventional methods, as described e.g. in Remington's Pharmaceutical Sciences, 19th Edition (Mack Publishing Company, 1995), incorporated herein by reference.

In some embodiments, the pharmaceutical composition comprises from 5 mg to 1,200 mg of lanifibranor. Exemplary pharmaceutical compositions comprise 5 mg, 10 mg, 15 mg, 20 mg, 25 mg, 30 mg, 35 mg, 40 mg, 45 mg, 50 mg, 55 mg, 60 mg, at 65mg, 70 mg, 75 mg, 80 mg, 85 mg, 90 mg, 95 mg, 100 mg, 150 mg, 200 mg, 250 mg, 300 mg, 350 mg, 400 mg, 450 mg, 500 mg, 550 mg, 600 mg, 650 mg, 700 mg, 750 mg, 800 mg, 850 mg, 900 mg, 950 mg, 1,000 mg, 1,050 mg, 1,100 mg, 1,150 mg, or 1,200 mg of lanifibranor.

In some embodiments, the pharmaceutical composition is administered via oral, parenteral or topical route.

In some embodiments, crystalline form beta of lanifibranor or a pharmaceutical composition comprising crystalline form beta of lanifibranor can be used in a method for the prevention and/or treatment of a liver disease in a subject in need thereof. In some embodiments, the liver disease is selected from cirrhosis, liver fibrosis, liver steatosis, fatty liver disease, acute decompensation, acute on chronic liver failure (ACFL), acute liver failure (ALF), decompensated cirrhosis, non-alcoholic fatty liver disease (NAFLD), non-alcoholic steatohepatitis (NASH), metabolic dysfunction-associated fatty liver disease (MAFLD), metabolic dysfunction-associated steatohepatitis (MASH), liver cirrhosis, non-alcoholic steatohepatitis with moderate to advanced liver fibrosis, metabolic dysfunction-associated steatohepatitis with moderate to advanced liver fibrosis, noncirrhotic non-alcoholic steatohepatitis with moderate to advanced liver fibrosis, noncirrhotic metabolic dysfunction-associated steatohepatitis with moderate to advanced liver fibrosis. In some embodiments, the said moderate to advanced liver fibrosis is selected from stages F2 to F3 fibrosis. In some embodiments, crystalline form beta of lanifibranor or a pharmaceutical composition comprising crystalline form beta of lanifibranor can be used in a method for treating a cirrhotic subject at risk of progressing from compensated stage to decompensated stage. In some embodiments, crystalline form beta of lanifibranor or a pharmaceutical composition comprising crystalline form beta of lanifibranor can be used in a method for treating a subject, wherein the subject has decompensated cirrhosis or is at risk of decompensated cirrhosis or acute decompensation. In some embodiments, crystalline form beta of lanifibranor or a pharmaceutical composition comprising crystalline form beta of lanifibranor can be used in a method for the reversion of decompensated cirrhosis to compensated cirrhosis.

In some embodiments, the present invention is directed to a method for the prevention and/or treatment of a liver disease in a subject in need thereof, comprising administering to said subject a therapeutically amount of the crystalline form beta of lanifibranor or a pharmaceutical composition comprising crystalline form beta of lanifibranor. In some embodiments, the liver disease is selected from cirrhosis, liver fibrosis, liver steatosis, fatty liver disease, acute decompensation, acute on chronic liver failure (ACFL), acute liver failure (ALF), decompensated cirrhosis, non-alcoholic fatty liver disease (NAFLD), non-alcoholic steatohepatitis (NASH), metabolic dysfunction-associated fatty liver disease (MAFLD), metabolic dysfunction-associated steatohepatitis (MASH), liver cirrhosis, non-alcoholic steatohepatitis with moderate to advanced liver fibrosis, metabolic dysfunction-associated steatohepatitis with moderate to advanced liver fibrosis, noncirrhotic non-alcoholic steatohepatitis with moderate to advanced liver fibrosis, noncirrhotic metabolic dysfunction-associated steatohepatitis with moderate to advanced liver fibrosis. In some embodiments, the said moderate to advanced liver fibrosis is selected from stages F2 to F3 fibrosis. In some embodiments, the present invention is directed to a method for treating a cirrhotic subject at risk of progressing from compensated stage to decompensated stage, comprising administering to said subject a therapeutically amount of the crystalline form beta of lanifibranor or a pharmaceutical composition comprising crystalline form beta of lanifibranor. In some embodiments, the subject has decompensated cirrhosis or is at risk of decompensated cirrhosis or acute decompensation. In some embodiments, the present invention is directed to a method for treating the reversion of decompensated cirrhosis to compensated cirrhosis, comprising administering to said subject a therapeutically amount of the crystalline form beta of lanifibranor or a pharmaceutical composition comprising crystalline form beta of lanifibranor. The present disclosure is illustrated by the following examples.

### Abbreviations

CuI = copper iodide
eq = equivalent
DMF = N,N-dimethylformamide
h = hour
iPrOH = isopropanol
MeTHF = 2-methyl tetrahydrofuran
Min = minute
NAC = N-acetyl-L-cysteine
NEt₃ = triethylamine
Pd/C = palladium-on-carbon
RT = room temperature
THF = tetrahydrofuran
V = volume
1 volume of acetic acid = 250 mL
1 volume of water = 250 mL

### Methods

XRPDs of the samples were measured using D8 Advance Bruker AXS diffractometer. Measuring conditions: Cu Ka radiation, Ni filtered, Bragg-Brentano reflexion geometry; Si single-crystal specimen holder, 2,5° linear detector 'LynxEye', step size: D2Q 0.02°, measuring time: 1s/step. The Raman spectrum of the crystalline form beta of lanifibranor was obtained using a MacroRAM^{™} spectrometer available from Horiba. The laser used for the measurement had a wavelength of 785 nm. The lanifibranor sample was pulverized in fine powder and placed on the sample device as a few-millimeter spot.

Powder flow and the angle of repose were determined using an Electrolab EFT-01 apparatus. In a first step, powder flow is measured through various orifices (10, 15 and 25 mm). In a second step, the angle of repose is measured by the height of the cone formed by a sample of 50 g of powder passing through an orifice of 25 mm. The result obtained in the rounded average of three experiments.

The compressibility index and Hausner ratio were determined using an Electrolab ETD-1020 apparatus using measured values of bulk density (ρ*bulk*) and tapped density (ρ*tapped*) as follows: $Compressibility Index = 100 \times \frac{ρ tapped - ρ bulk}{ρ tapped}$ $Hausner Ratio = \frac{ρtapped}{ρ bulk}$

### Example 1

N-(4-chloro-2-iodophenyl)benzothiazole-6-sulfonamide (200.0 g, 1 eq, 443.76 mmol) and CuI (7.6 g, 0.09 eq, 39.939 mmol) were placed in a 2 L-round-bottom flask. DMF (973.2 g, 1.0 L, 30 eq, 13.313 mol) was added to the reaction mixture. The reaction was warmed to 50°C. Afterwards, Pd/C 20% (7.0838 g, 20 wt%, 0.03 eq, 13.313 mmol) was added to the warm reaction mixture. NEt₃ (89.8 g, 124 mL, 2 eq, 887.53 mmol) and 5-hexynoic acid (73.329 g, 72.17 mL, 95 wt%, 1.4 eq, 621.27 mmol) were added within 30 min to the reaction mixture, which was warmed from 50°C to 75°C, and then stirred at 75°C for 2 h. The catalyst was filtered and DMF was distilled off (15-20 mbar, 60°C). The reaction mixture was diluted with 6 V of 2-MeTHF and 2 V of water, and HCl 30% was then added to the reaction mixture to pH 1-2. The organic phase was washed 4 times with 0.2 eq. of a 6% NAC solution and then twice with 2 V of water. The organic phase was mixed for 30 min at 50°C with 5 wt% of Norit Supra A, filtered and washed with 0.5 V of Me-THF. The organic phase was then concentrated and cooled down to RT. 1 V of iPrOH was added, the mixture was stirred for 40 min, filtrated and washed with 0.5 V:0.5 V of an iPrOH:water mixture. The product was dried in a vacuum oven at 45°C/20 mbar overnight to give 152.35 g of lanifibranor in 80% yield with a purity of 99.88% (as determined by HPLC).

APCI MS m/z 435 [M+H]+; UPLC-MS (210-260 nm) purity: 99.9%.

¹H NMR (300 MHz, DMSO): δ 12.13 (s, 1H), 9.66 (s, 1H), 8.98 (d, J = 2.0 Hz, 1H), 8.20 (d, J = 8.7 Hz, 1H), 8.09 (d, J = 8.9 Hz, 1H), 7.85 (dd, J = 8.7, 2.1 Hz, 1H), 7.57 (d, J = 2.2 Hz, 1H), 7.32 (dd, J = 8.9, 2.2 Hz, 1H), 6.62 (d, J = 1.0 Hz, 1H), 3.09 (t, J = 7.5 Hz, 2H), 2.36 (t, J = 7.3 Hz, 2H), 1.94 (q, J = 7.5 Hz, 2H).

¹³C NMR (75 MHz, DMSO): δ 174.60, 163.06, 156.62, 143.53, 135.21, 135.17, 134.38, 131.45, 128.77, 124.70, 124.43, 123.91, 123.38, 120.43, 116.22, 109.18, 33.44, 28.16, 24.07.

### Example 2

N-(4-chloro-2-iodophenyl)benzo[d]thiazole-6-sulfonamide (10.0 g, 1 eq., 22.19 mmol), CuI (380.32 mg, 0.09 eq., 2 mmol), and Pd/C 20% (2 x 354.19 mg, 20% wt., 0.03 eq., 665.65 µmol, a double amount of Pd/C 20% was used (708 mg), as it is 50% wet) were placed in a 250 mL-round-bottom flask. DMF (48.6 g, 50 mL, 30 eq., 665.65 mmol) and water (10 g, 10.0 mL, 25 eq., 554.71 mmol) were added to the reaction mixture, which was warmed up to 50°C. NEt₃ (4.5 g, 6.2 mL, 2 eq., 44.38 mmol) and hex-5-ynoic acid (3.5 g, 3.43 mL, 1.4 eq., 31.1 mmol) were added simultaneously within 30 minutes to the reaction mixture, which was warmed from 50°C to 75°C within this time. The mixture was later stirred at 75°C for 1 h.

After the reaction was over the catalyst was filtered off. DMF and water were distilled off (85-95% recovery) (15-20 mbar, 60°C bath temperature). The reaction mixture was diluted with 6 V of Me-THF and 2 V of water. HCl 30% was added to the reaction mixture to pH 1-2. The organic phase and the water phase were stirred and mixed, afterwards the water phase was removed. The organic phase was then washed 4 times with 0.2 eq. of 6 % solution of N-Acetyl-L-(+)-cysteine (NAC) (12.07 g, 6 % wt., 0.2 eq., 4.44 mmol) by stirring at 50°C for 30 minutes and twice with 2 V each of water by stirring at 50°C for 30 minutes.

The organic phase was stirred for 30 minutes at 50°C with charcoal (Norit Supra A, 5% wt.), filtered and washed with 0.5 V of Me-THF. The organic phase was then concentrated to dryness.

1 V of iPrOH and 1 V of water were added and the mixture was stirred for 40 minutes, filtered and washed with 0.5 V:0.5 V of an iPrOH:water mixture. The product was dried in vacuum oven at 45°C/20 mbar overnight to give 8.77 g of lanifibranor in 88.7% yield with a purity of 99.64% (as determined by HPLC).

### Example 3

The same process as described in Example 2 was used with a different amount of N-(4-chloro-2-iodophenyl)benzo[d]thiazole-6-sulfonamide (17.0 g, 1 eq., 37.72 mmol). 14.95 g of lanifibranor were thus obtained in 88.6% yield with a purity of 99.68% (as determined by HPLC).

### Example 4

Lanifibranor as obtained in example 1 (250 g, 573 mmol) was placed into a 3-neck flask and acetic acid (1.6 kg, 1.5 L, 46 eq, 26 mol) was added thereto. The mixture was warmed up to 105°C and stirred until a slurry of lanifibranor was obtained. 12.5 g (5 wt%) of Norit^{™} Supra A active charcoal was added to the reaction mixture which was then filtered over a Seitz K300 filter at 100-105°C and rinsed 2 times with 0.2 V of acetic acid. The solution was concentrated to 3.6 V of acetic acid (105°C/650 mbar) and 0.4 V of water (100 g, 100 mL, 9.7 eq, 5.56 mol) was added within 30 min at 100-105°C. The mixture was cooled to 60°C and stirred at this temperature for 16h. The mixture was then allowed to return to RT, filtered and washed with 0.5 V (125 mL) of acetic acid and 3 times with 1 V (250 mL) of water. The product was dried at 60°C/20 mbar overnight to give a solid form of lanifibranor in 94% yield and 100% purity (as determined by HPLC). The PXRD pattern of the solid form obtained is in accordance with figure 1; the DSC curve of the solid form obtained is in accordance with figure 2; the TGA curve of the solid form obtained is in accordance with figure 3. This establishes that said solid form is crystalline form beta of lanifibranor.

### Example 5

Lanifibranor as obtained in example 1 was placed in acetic acid. The mixture was maintained under constant stirring at room temperature for 2 hours. After the stirring period, the solid was isolated by filtration.

The PXRD pattern of the solid form obtained is in accordance with figure 1; the DSC curve of the solid form obtained is in accordance with figure 2; the TGA curve of the solid form obtained is in accordance with figure 3. This establishes that said solid form is crystalline form beta of lanifibranor.

### Example 6

Lanifibranor as obtained in example 1 was placed in ethanol. The mixture was maintained under constant stirring at room temperature for 4 hours. After the stirring period, the solid was isolated by filtration.

The PXRD pattern of the solid form obtained is in accordance with figure 1; the DSC curve of the solid form obtained is in accordance with figure 2; the TGA curve of the solid form obtained is in accordance with figure 3. This establishes that said solid form is crystalline form beta of lanifibranor.

### Example 7

Lanifibranor as obtained in example 1 was placed in ethyl acetate. The mixture was maintained under constant stirring at room temperature for 3 hours. After the stirring period, the solid was isolated by filtration.

The PXRD pattern of the solid form obtained is in accordance with figure 1; the DSC curve of the solid form obtained is in accordance with figure 2; the TGA curve of the solid form obtained is in accordance with figure 3. This establishes that said solid form is crystalline form beta of lanifibranor.

### Example 8

Lanifibranor as obtained in example 1 was placed in water. The mixture was maintained under constant stirring at room temperature for 1 day (24 hours). After the stirring period, the solid was isolated by filtration.

The PXRD pattern of the solid form obtained is in accordance with figure 1; the DSC curve of the solid form obtained is in accordance with figure 2; the TGA curve of the solid form obtained is in accordance with figure 3. This establishes that said solid form is crystalline form beta of lanifibranor.

### Exemple 9

Lanifibranor as obtained in example 1 was placed in 10 mL glass vial with a PTFE stirrer. 4 mL of acetic acid were added thereto. The reaction was stirred for 16 h at 400 rpm at 60 °C. The mixture was filtered, washed with 0.5 mL of acetic acid and then 3 times with 1 mL of water, and dried at 60 °C/20 mbar. The PXRD pattern of the solid form obtained is in accordance with figure 1; the DSC curve of the solid form obtained is in accordance with figure 2; the TGA curve of the solid form obtained is in accordance with figure 3. This establishes that said solid form is crystalline form beta of lanifibranor.

### Example 10

Lanifibranor as obtained in example 1 (250 g, 573 mmol) was placed into a 3-neck flask and THF/toluene (1.6 kg, 1.5 L, 46 eq, 26 mol) was added thereto. The mixture was warmed up to 105°C and stirred until a slurry of lanifibranor was obtained. 12.5 g (5 wt%) of Norit Supra A active charcoal was added to the reaction mixture which was then filtered over a Seitz K300 filter at 100-105°C and rinsed twice with 0.2 V of THF/toluene. The solution was concentrated to 3.6 V of THF/toluene (105°C/650 mbar) and 0.4 V of water (100 g, 100 mL, 9.7 eq, 5.56 mol) was added within 30 min at 100-105°C. The mixture was cooled to 65°C and stirred at this temperature for 16h. The mixture was then allowed to return to RT, filtered and washed with 0.5 V (125 mL) of THF/toluene and 3 times with 1 V (250 mL) of water. The product was dried at 60°C/20 mbar overnight to give a solid form of lanifibranor in 67% yield and 100% purity (as determined by HPLC). The PXRD pattern of the solid form obtained is in accordance with figure 1; the DSC curve of the solid form obtained is in accordance with figure 2; the TGA curve of the solid form obtained is in accordance with figure 3. This establishes that said solid form is crystalline form beta of lanifibranor.

### Example 11

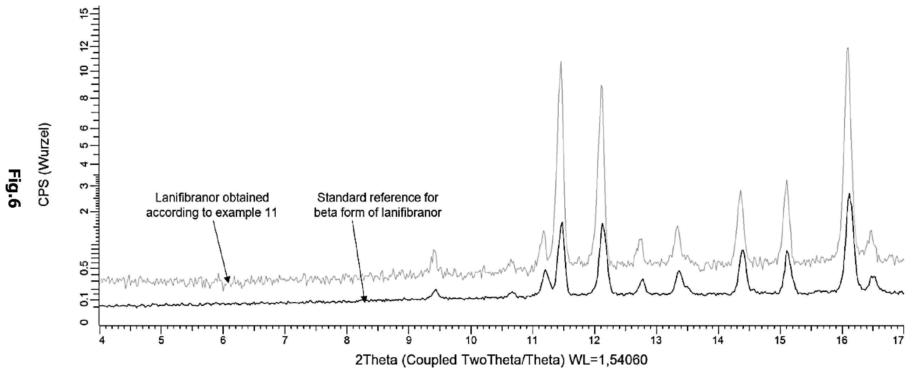

1 g of lanifibranor as obtained in example 1 was placed in a 10 mL glass vial with a PTFE stirrer. 4 mL of acetic acid were added thereto. The mixture was stirred for 16h at 400 rpm at 60°C and then filtered. The solid was washed with 0.5 mL of acetic acid, then 3 times with 1 mL of water and dried at 60°C/20 mbar. PXRD analysis of the solid formed obtained was performed. It can be seen from Figure 6 that the PXRD pattern of the solid form obtained superimposes with the PXRD pattern as shown in Figure 1.

### Example 12

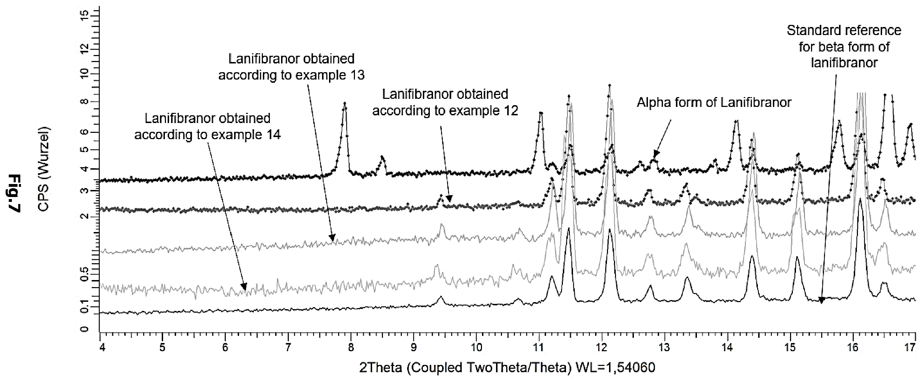

1 g of lanifibranor as obtained in example 1 was placed in a 10 mL glass vial with a PTFE stirrer. 4 mL of acetic acid was added thereto. The slurry of lanifibranor was seeded with 20 mg (2 wt.%) of pure beta form of lanifibranor. The mixture was stirred for 16 h at 400 rpm at 50°C and then filtered. The solid was washed with 0.5 mL of acetic acid, then 3 times with 1 mL of water and dried at 60 °C/20 mbar. PXRD analysis of the solid formed obtained was performed. It can be seen from Figure 7 that the PXRD pattern of the solid form obtained superimposes with the PXRD pattern as shown in Figure 1.

### Example 13

1 g of lanifibranor as obtained in example 1 was placed in a 10 mL glass vial with a PTFE stirrer. 4 mL of acetic acid was added thereto. The slurry of lanifibranor was seeded with 20 mg (2 wt.%) of pure beta form of lanifibranor. The mixture was stirred for 16 h at 400 rpm at 60°C and then filtered. The solid was washed with 0.5 mL of acetic acid, then 3 times with 1 mL of water, and dried at 60 °C/20 mbar. PXRD analysis of the solid formed obtained was performed. It can be seen from Figure 7 that the PXRD pattern of the solid form obtained superimposes with the PXRD pattern as shown in Figure 1.

### Example 14

1 g of lanifibranor as obtained in example 1 was placed in a 10 mL glass vial with a PTFE stirrer. 4 mL of acetic acid was added thereto. The slurry of lanifibranor was seeded with 20 mg (2 wt.%) of pure beta form of lanifibranor. The mixture was stirred for 16 h at 400 rpm at 80°C and then filtered. The solid was washed with 0.5 mL of acetic acid, then 3 times with 1 mL of water and dried at 60 °C/20 mbar. PXRD analysis of the solid formed obtained was performed. It can be seen from Figure 7 that the PXRD pattern of the solid form obtained superimposes with the PXRD pattern as shown in Figure 1.

### Example 15

The angle of repose, compressibility index and Hausner ratio of crystalline form beta of lanifibranor as obtained in example 2 were determined according to the methods described above. The angle of repose was 35°, the compressibility index was 20.64 and the Hausner ratio was 1.26.

### Example 16

The same process as in example 2 was used, with a different amount of water (0.3 V vs 1V) and a final stirring time (to complete the reaction and before the filtration of the catalyst) of 2h at 75°C vs 1h at 75°C. Lanifibranor was obtained in 81% yield with a purity of 98.75% (as determined by HPLC).

### Example 17

N-(4-chloro-2-iodophenyl)benzo[d]thiazole-6-sulfonamide (20.0 g, 1 eq., 44.38 mmol), copper(I) iodide (380 mg, 0.045 eq., 2 mmol), Pd/C 20% (354 mg, 20% wt., 0.015 eq., 665.65 µmol, a double amount of Pd/C 20% was used (708 mg), as it is 50% wet) were placed in a 250 mL-round-bottom 2-necked flask, which was then purged with argon. The atmosphere purge with argon was repeated twice. DMF (38 g, 40.0 mL, 11.65 eq., 516.99 mmol) and water (39.9 g, 40.0 mL, 49.9 eq., 2.21 mol) were mixed together and degassed with argon for 20-30 min. The degassed solvents were then added to the reaction mixture under argon and the mixture was warmed up to 50°C. Hex-5-ynoic acid (7.41 g, 7.294 mL, 94% wt, 1.4 eq., 62.13 mmol) and NEt₃ (9 g, 12.4 mL, 2 eq., 88.75 mmol) were added simultaneously to the mixture over 30 min, during which time the reaction was heated from 50°C to 75°C. The mixture was further stirred at 75°C for 1 h.

After the reaction was over the catalyst was filtered off. DMF and water were distilled off (85-95% recovery). The reaction mixture was diluted with 6 V of Me-THF and 1.5 V of water. HCl 30% was added to the reaction mixture to pH 1-2. The organic and aqueous phases were stirred and mixed, and the aqueous phase was removed. The organic phase was then washed 4 times with a 6% solution of NAC (24.14 g, 6% wt., 0.2 eq., 8.87 mmol) by stirring at 60°C for 30 minutes and twice with 2 V of water by stirring at 60°C for 30 minutes.

The reaction mixture was concentrated to 2 V of Me-THF, then 2 V of acetic acid were added to the mixture. A slow and continuous addition of acetic acid was continued while distilling off the mixture of Me-THF and acetic acid at 200 mbar until a 71°C head temperature was reached. The product obtained was used as such in the next example.

### Example 18

The product obtained in example 17 was dissolved in acetic acid (89 g, 85 mL, 33.5 eq., 1.49 mol) (overall volume, 4.4 V, calculated on 100% yield of lanifibranor) by heating the mixture to 105°C. 5% of charcoal (Norit^{™} Supra A) was added to the mixture which was stirred for 30 min. The activated charcoal was filtered over a GL filter, washed twice with 1 V of acetic acid and the filtrate was concentrated to 3.6 V (calculated on 100% yield of lanifibranor) by distillation at 105°C. At this temperature water (7.7 g, 7.7 mL, 9.6 eq., 426 mmol) was added to the reaction mixture over 30 min. During the addition the temperature was decreased to 100°C whereby a product precipitated. The mixture was stirred at 90°C for 1 h, and subsequently cooled to 60°C over 4 hours and stirred at this temperature overnight. The reaction mixture was cooled down to 20°C over 1 h and was left under stirring for 4 h. It was then filtered, washed with 0.5 V of acetic acid and three times with 1 V of water. The product was dried 3-4 h at 45°C in a vacuum drying oven, to give a solid form of lanifibranor (16.61 g, 38.23 mmol, 86.14 %).

¹H NMR (300 MHz, DMSO): δ 12.20 (s, 1H), 9.62 (s, 1H), 8.96 (d, J = 2.0 Hz, 1H), 8.17 (d, J = 8.7 Hz, 1H), 8.09 (d, J = 8.9 Hz, 1H), 7.85 (dd, J = 8.7, 2.0 Hz, 1H), 7.47 (d, J = 2.2 Hz, 1H), 7.26 (dd, J = 8.9, 2.2 Hz, 1H), 6.55 (s, 1H), 3.10 (t, J = 7.5 Hz, 2H), 2.39 (t, J = 7.3 Hz, 2H), 1.95 (p, J = 7.5 Hz, 2H).

¹³C NMR (75 MHz, DMSO): δ 174.68, 162.89, 156.59, 143.49, 135.19, 135.13, 134.40, 131.40, 128.78, 124.65, 124.38, 123.90, 123.32, 120.34, 116.17, 109.09, 33.47, 28.20, 24.04.

It was confirmed by PXRD that the solid form obtained was crystalline form beta of lanifibranor. The solid form obtained was also characterized by Raman analysis, with peaks as listed in table 2 and shown in Figure 10.

**Table 2**

| Position (cm⁻¹) | Intensity (counts) |
|---|---|
| 117.2 | 1119.0 |
| 146.2 | 546.3 |
| 161.2 | 335.8 |
| 185.5 | 231.3 |
| 212.5 | 204.5 |
| 233.4 | 732.0 |
| 250.2 | 261.9 |
| 275.3 | 761.0 |
| 307.9 | 233.4 |
| 334.5 | 182.4 |
| 348.2 | 57.7 |
| 373.2 | 305.3 |
| 396.3 | 152.2 |
| 410.7 | 103.9 |
| 437.5 | 62.1 |
| 448.4 | 168.4 |
| 506.9 | 73.1 |
| 520.4 | 117.5 |
| 532.8 | 353.8 |
| 554.8 | 136.3 |
| 568.8 | 510.8 |
| 586.9 | 169.9 |
| 621.2 | 212.5 |
| 646.8 | 355.6 |
| 690.8 | 221.7 |
| 702.3 | 121.6 |
| 720.2 | 700.2 |
| 741.7 | 124.3 |
| 767.3 | 449.3 |
| 804.3 | 89.0 |
| 822.5 | 294.3 |
| 855.1 | 154.2 |
| 901.5 | 49.2 |
| 929.3 | 203.7 |
| 1040.4 | 283.1 |
| 1063.2 | 83.8 |
| 1078.2 | 184.0 |
| 1103.8 | 139.4 |
| 1134.0 | 131.4 |
| 1142.4 | 134.8 |
| 1179.6 | 395.9 |
| 1208.5 | 140.3 |
| 1217.3 | 144.9 |
| 1242.0 | 244.6 |
| 1277.5 | 227.4 |
| 1307.2 | 176.0 |
| 1327.8 | 230.7 |
| 1362.0 | 61.3 |
| 1410.1 | 63.4 |
| 1435.2 | 174.9 |
| 1443.5 | 158.8 |
| 1465.4 | 125.5 |
| 1560.7 | 376.6 |
| 1586.8 | 197.5 |

Aspects of the present disclosure are further illustrated by reference to the following nonlimiting embodiments.
1. A process for the manufacture of lanifibranor, said process comprising reacting N-(4-chloro-2-iodo-phenyl)-1,3-benzothiazole-6-sulfonamide with 5-hexynoic acid in the presence of Pd/C and of a copper salt.
2. The process of item 1, wherein the copper salt is selected from copper chloride, copper acetate, copper carbonate, copper sulfate, copper bromide, trifluoroacetic acid copper, copper nitrate, copper hydroxide, copper oxide, copper iodide and mixtures thereof.
3. The process of item 2, wherein the copper salt is copper iodide.
4. The process of any preceding item, wherein the copper salt is used in an amount from about 0.01 to about 0.15 equivalent, based on the amount of starting N-(4-chloro-2-iodophenyl)-1,3-benzothiazole-6-sulfonamide.
5. The process of any preceding item, wherein 5-hexynoic acid is used in an amount from about 1.0 to about 2.0 equivalents, based on the amount of starting N-(4-chloro-2-iodophenyl)-1,3-benzothiazole-6-sulfonamide.
6. The process of any preceding item, wherein the reaction is carried out in a solvent.
7. The process of item 6, wherein said solvent is selected from dichloromethane, chloroform, pyridine, 4-dimethylaminopyridine, N,N-dimethylformamide, dimethyl sulfoxide, acetonitrile, tetrahydrofuran, 2-methyl tetrahydrofuran, water, ethyl acetate, toluene, acetone, methylethylketone, methylisobutylketone, cyclohexanone, methylbutylketone and mixtures thereof.
8. The process of item 7, wherein said solvent is selected from N,N-dimethylformamide, tetrahydrofuran, water and mixtures thereof.
9. The process of any preceding item, wherein the reaction is carried out in the presence of a base.
10. The process of item 9, wherein the base is selected from trimethylamine, triethylamine, pyridine, imidazole, 4-dimethylaminopyridine, 2,6-lutidine, 1,4-diazabicyclo[2.2.2]octane, 1,8-diazabicyclo[5.4.0]undec-7-ene, 1,5-diazabicyclo[4.3.0]non-5-ene, N,N-dimethylphenyl-amine and mixtures thereof.
11. The process of item 9 or item 10, wherein the base is used in an amount of about 1.0 to about 20.0 equivalents, based on the amount of starting N-(4-chloro-2-iodo-phenyl)-1,3-benzothiazole-6-sulfonamide.
12. The process of any preceding item, wherein the reaction is carried out at a temperature from about 35°C to about 200°C.
13. A process for the manufacture of crystalline form beta of lanifibranor, comprising:
   a) preparing a hot slurry of lanifibranor in a suitable solvent for lanifibranor;
   b) optionally, filtering the hot slurry of lanifibranor;
   c) cooling the slurry of lanifibranor to a temperature from about 15°C to about 95°C; and
   d) maintaining the slurry of lanifibranor to a temperature from about 15°C to about 95°C for about 15 minutes to about 72h.
14. The process of item 13, wherein the suitable solvent for lanifibranor is selected from water, dimethyl sulfoxide, alcohol solvents, halogenated hydrocarbons solvents, ether solvents, ketone solvents, esters solvents, hydrocarbons solvents, nitrile solvents, amide solvents and mixtures thereof.
15. The process of item 14, wherein the suitable solvent for lanifibranor is selected from toluene, tetrahydrofuran, acetic acid and mixtures thereof.
16. The process of any one of items 13 to 15, wherein the slurry of lanifibranor is heated in step a) to a temperature from about 70°C to about 120°C.
17. The process of item 16, wherein the slurry of lanifibranor is heated in step a) to a temperature from about 70°C to about 110°C, from about 80°C to about 105°C, or from about 85°C to about 100°C.
18. The process of any one of items 13 to 17, wherein the slurry of lanifibranor is cooled in step c) to a temperature in the range from 95°C to about 15°C.
19. The process of item 18, wherein the slurry of lanifibranor is cooled in step c) to a temperature in the range from about 95°C to about 20°C, from about 95°C to about 25°C, from about 95°C to about 30°C, from about 95°C to about 35°C, from about 95°C to about 40°C, from about 95°C to about 45°C, from about 95°C to about 50°C, from about 95°C to about 55°C, from about 95°C to about 60°C, from about 95°C to about 65°C, from about 95°C to about 70°C, from about 95°C to about 75°C, from about 95°C to about 80°C, from about 95°C to about 85°C, or from about 95°C to about 90°C.
20. The process of any one of items 13 to 19, wherein the slurry of lanifibranor is cooled in step c) over a period of time from about 30 min to about 15 hours.
21. The process of item 20, wherein the slurry of lanifibranor is cooled in step c) over a period of time from about 30 min to about 15 hours, from about 30 min to about 14 hours, from about 30 min to about 13 hours, from about 30 min to about 12 hours, or from about 30 min to about 11 hours, from about 30 min to about 10 hours, from about 30 min to about 3 hours, from about 30 min to about 8 hours, or from about 30 min to about 7 hours, from about 30 min to about 6 hours, from about 30 min to about 5 hours, from about 30 min to about 4 hours, from about 30 min to about 3 hours, from about 30 min to about 2 hours, or from about 30 min to about 1 hour.
22. The process of any one of items 13 to 21, wherein the slurry of lanifibranor is maintained in step d) at a temperature from about 95°C to about 15°C.
23. The process of item 22, wherein the slurry of lanifibranor is maintained in step d) at a temperature in the range from about 95°C to about 20°C, from about 95°C to about 25°C, from about 95°C to about 30°C, from about 95°C to about 35°C, from about 95°C to about 40°C, from about 95°C to about 45°C, from about 95°C to about 50°C, from about 95°C to about 55°C, from about 95°C to about 60°C, from about 95°C to about 65°C, from about 95°C to about 70°C, from about 95°C to about 75°C, from about 95°C to about 80°C, from about 95°C to about 85°C, or from about 95°C to about 90°C.
24. The process of any one of items 13 to 23, wherein step d) is carried out for a period of time of about 15 minutes to about 72 hours.
25. The process of item 24, wherein step d) is carried out for a period of time of about 30 minutes to about 48 hours, about 1 hour to about 30 hours, about 1 hour to 25 hours, about 1 hour to 20 hours, about 1 hour to 15 hours, or about 1 hour to about 10 hours.
26. The process of any one of items 13 to 25, wherein the slurry of lanifibranor is maintained in step d) at the temperature at which it has been cooled down in step c).
27. The process of any one of items 13 to 26, wherein no seeds of crystalline form beta of lanifibranor are added during the reaction.
28. The process of any one of items 13 to 26, wherein seeds of crystalline form beta of lanifibranor are added during step c) and/or step d).
29. The process of any one of items 13 to 28, wherein, steps c) and d) are repeated from one to 10 times.
30. Crystalline form beta of lanifibranor obtained by the process of any one of items 13 to 29, wherein said crystalline form has an angle of repose comprised between about 33° and about 37°.
31. The crystalline form beta of lanifibranor of item 30, which has a compressibility index of about 16 to about 25, and a Hausner ratio of about 1.19 to about 1.34.
32. A pharmaceutical composition comprising the crystalline form beta of lanifibranor of any of items 30 to 31 and at least one pharmaceutically acceptable carrier or excipient.
33. Crystalline form beta of lanifibranor of one of items 30 to 31, or pharmaceutical composition of item 32, for use in a method for the prevention and/or treatment of a liver disease.
34. Crystalline form beta of lanifibranor of one of items 30 to 31, or pharmaceutical composition of item 32, for use in a method for the prevention and/or treatment of a liver disease selected among cirrhosis, liver fibrosis, liver steatosis, fatty liver disease, acute decompensation, acute on chronic liver failure, acute liver failure, decompensated cirrhosis, non-alcoholic fatty liver disease, non-alcoholic steatohepatitis, metabolic dysfunction-associated fatty liver disease, metabolic dysfunction-associated steatohepatitis, liver cirrhosis, non-alcoholic steatohepatitis with moderate to advanced liver fibrosis, metabolic dysfunction-associated steatohepatitis with moderate to advanced liver fibrosis, noncirrhotic non-alcoholic steatohepatitis with moderate to advanced liver fibrosis, noncirrhotic metabolic dysfunction-associated steatohepatitis with moderate to advanced liver fibrosis.
35. Crystalline form beta of lanifibranor of one of items 30 to 31, or pharmaceutical composition of item 32, for use in a method for the reversion of decompensated cirrhosis to compensated cirrhosis.
36. Crystalline form beta of lanifibranor of one of items 30 to 31, or pharmaceutical composition of item 32, for use in a method of treating a cirrhotic subject at risk of progressing from compensated stage to decompensated stage.
37. A method of preventing and/or treating a liver disease selected among cirrhosis, liver fibrosis, liver steatosis, fatty liver disease, acute decompensation, acute on chronic liver failure, acute liver failure, decompensated cirrhosis, non-alcoholic fatty liver disease, non-alcoholic steatohepatitis, metabolic dysfunction-associated fatty liver disease, metabolic dysfunction-associated steatohepatitis, liver cirrhosis, non-alcoholic steatohepatitis with moderate to advanced liver fibrosis, metabolic dysfunction-associated steatohepatitis with moderate to advanced liver fibrosis, noncirrhotic non-alcoholic steatohepatitis with moderate to advanced liver fibrosis, and noncirrhotic metabolic dysfunction-associated steatohepatitis with moderate to advanced liver fibrosis, which comprises administering to a subject in need thereof an effective amount of a crystalline form beta of lanifibranor as defined in one of items 30 to 31, or a pharmaceutical composition as defined in item 32.
35. A method of reverting decompensated cirrhosis to compensated cirrhosis, which comprises administering to a subject in need thereof an effective amount of a crystalline form beta of lanifibranor as defined in one of items 30 to 31, or a pharmaceutical composition as defined in item 32.
36. A method of treating a cirrhotic subject at risk of progressing from compensated stage to decompensated stage which comprises administering to the subject an effective amount of a crystalline form beta of lanifibranor as defined in one of items 30 to 31, or a pharmaceutical composition as defined in item 32.

## Claims

1. A process for the manufacture of crystalline form beta of lanifibranor, wherein said crystalline form has a PXRD pattern comprising peaks at 16.0° ± 0.2°, 18.7° ± 0.2° and 24.6° ± 0.2° 2θ, as measured using an X-ray wavelength of 1.5406 Å, the said process comprising:
a) preparing a hot slurry of lanifibranor in a suitable solvent for lanifibranor;
b) optionally, filtering the hot slurry of lanifibranor;
c) cooling the slurry of lanifibranor to a temperature from 15°C to 95°C; and
d) maintaining the slurry of lanifibranor to a temperature from 15°C to 95°C for 15 minutes to 72h.

2. The process of claim 1, wherein the suitable solvent for lanifibranor is selected from water, dimethyl sulfoxide, alcohol solvents, halogenated hydrocarbons solvents, ether solvents, ketone solvents, esters solvents, hydrocarbons solvents, nitrile solvents, amide solvents and mixtures thereof.

3. The process of claim 2, wherein the suitable solvent for lanifibranor is selected from toluene, tetrahydrofuran, acetic acid and mixtures thereof.

4. The process of claim 1 or claim 3, wherein the slurry of lanifibranor is heated in step a) to a temperature from 70°C to 120°C, such as for example from 70°C to 110°C, from 80°C to 105°C, or from 85°C to 100°C.

5. The process of any one of claims 1 to 4, wherein the slurry of lanifibranor is cooled in step c) to a temperature from 15°C to 95°C, from 20°C to 95°C, from 25°C to 95°C, from 30°C to 95°C, from 35°C to 95°C, from 40°C to 95°C, from 45°C to 95°C, from 50°C to 95°C, from 55°C to 95°C, from 60°C to 95°C, from 65°C to 95°C, from 70°C to 95°C, from 75°C to 95°C, from 80°C to 95°C, from 85°C to 95°C, or from 90°C to 95°C.

6. The process of any one of claims 1 to 5, wherein the slurry of lanifibranor is maintained in step d) at a temperature from 15°C to 95°C, from 20°C to 95°C, from 25°C to 95°C, from 30°C to 95°C, from 35°C to 95°C, from 40°C to 95°C, from 45°C to 95°C, from 50°C to 95°C, from 55°C to 95°C, from 60°C to 95°C, from 65°C to 95°C, from 70°C to 95°C, from 75°C to 95°C, from 80°C to 95°C, from 85°C to 95°C or from 90°C to 95°C.

7. The process of any one of claims 1 to 6, wherein step d) is carried out for a period of time of 15 minutes to 72 hours, such as for example 30 minutes to 48 hours, 1 hour to 30 hours, 1 hour to 25 hours, 1 hour to 20 hours, or 1 hour to 15 hours.

8. The process of any one of claims 1 to 7, wherein the temperature in step d) is identical to the temperature in step c).

9. The process of any one of claims 1 to 8, wherein no seeds of crystalline form beta of lanifibranor are added during the reaction.

10. The process of any one of claims 1 to 8, wherein seeds of crystalline form beta of lanifibranor are added during step c) and/or step d).

11. The process of any one of claims 1 to 10, wherein steps c) and d) are repeated from one to ten times.

12. The process of claim 11, wherein steps c) and d) are repeated once or twice.

13. The process of claim 11 or claim 12, wherein steps c) and d) are repeated with different ranges of temperature.

14. A crystalline form beta of lanifibranor obtained by the process of any one of claims 1 to 13, wherein said crystalline form has an angle of repose comprised between 33° and 37°.

15. The crystalline form beta of lanifibranor of claim 14, which has a compressibility index of 16 to 25, and a Hausner ratio of 1.19 to 1.34.

16. A pharmaceutical composition comprising the crystalline form beta of lanifibranor of any of claims 14 to 15 and a pharmaceutically acceptable carrier or excipient.

17. The crystalline form beta of lanifibranor according to claims 14 to 15 or the pharmaceutical composition according to claim 16, for use in the prevention and/or treatment of a liver disease.

18. The crystalline form beta of lanifibranor or the pharmaceutical composition for the use according to claim 17, wherein the liver disease is selected among cirrhosis, liver fibrosis, liver steatosis, fatty liver disease, acute decompensation, acute on chronic liver failure, acute liver failure, decompensated cirrhosis, non-alcoholic fatty liver disease, non-alcoholic steatohepatitis, metabolic dysfunction-associated fatty liver disease, metabolic dysfunction-associated steatohepatitis, liver cirrhosis, non-alcoholic steatohepatitis with moderate to advanced liver fibrosis, metabolic dysfunction-associated steatohepatitis with moderate to advanced liver fibrosis, noncirrhotic non-alcoholic steatohepatitis with moderate to advanced liver fibrosis, noncirrhotic metabolic dysfunction-associated steatohepatitis with moderate to advanced liver fibrosis.

19. The crystalline form beta of lanifibranor according to claims 14 to 15 or the pharmaceutical composition according to claim 16 for use for the reversion of decompensated cirrhosis to compensated cirrhosis.

20. The crystalline form beta of lanifibranor according to claims 14 to 15 or the pharmaceutical composition according to claim 16, for use for treating a cirrhotic subject at risk of progressing from compensated stage to decompensated stage.
